# EUROPEAN PATENT APPLICATION

(11) **EP 4 736 783 A1**
(43) Date of publication of application: **06.05.2026**
(21) Application number: 25212563.8
(22) Date of filing: 31.10.2025
(51) Int. Cl.: A61B 17/02

(54) **CARDIAC RETRACTOR**

(30) Priority: 31.10.2024 US 202463714464 P
(71) Applicant: LSI Solutions, Inc., Victor, NY 14564 (US)
(72) Inventor: The designation of the inventor has not yet been filed
(74) Representative: Flügel Preissner Schober Seidel

(57) **Abstract**

A cardiac retractor (20, 152) is disclosed. The cardiac retractor (20, 152) has an outer tube (24, 162). The cardiac retractor (20, 152) also has a fixed collar (22, 165, 198) fixedly coupled to a proximal end (306) of the outer tube (24, 162). The cardiac retractor (20, 152) further has a fixed link (26) fixedly coupled to a distal end (160) of the outer tube (24, 162). The cardiac retractor (20, 152) also has an inner tube (98) rotatable within the outer tube (24, 162). The cardiac retractor (20, 152) further has a fixed key (118, 200) coupled to a proximal end (160) of the inner tube (98) and configured to rotate the inner tube (98) relative to the outer tube (24, 162). The cardiac retractor (20, 152) also has a keyed link (30) coupled to the distal end (160) of the outer tube (24, 162).

## Description

### CROSS-REFERENCE TO RELATED APPLICATIONS

This application claims priority to U.S. Provisional Patent Application No. 63/714,464, filed October 31, 2024, the contents of which is hereby incorporated by reference in its entirety. This application is also a continuation in part of U.S. Patent Application No. 17/395,631, filed August 6, 2021, which claims priority to U.S. Provisional Patent Application No. 63/066,376, filed August 17, 2020, the contents of each of which is hereby incorporated by reference in its entirety. In addition, U.S. Patent Application No. 17/395, 631 is a continuation-in-part of U.S. Patent Application No. 17/351,332, filed June 18, 2021, which is a continuation of U.S. Patent Application No. 16/407,171, filed May 8, 2019, which claims priority to U.S. Provisional Patent Application No. 62/668,751, filed May 8, 2018, the contents of each of which is hereby incorporated by reference in its entirety.

### FIELD

The claimed invention relates to surgical retractors, and more specifically to surgical retractors for use in minimally invasive cardiac surgery.

### BACKGROUND

Modern advances in cardiac surgery have made it possible to repair or replace heart valves using minimally invasive surgical techniques. As minimally invasive techniques have improved, surgeons are able to operate on patients through smaller and smaller access incisions, resulting in less perioperative pain and shorter recovery times. Unfortunately, many cardiac retractors used in maintaining an opening in the wall of the heart require more than one access incision when used in minimally invasive surgery: one incision for the majority of the retractor to be passed through, and a second incision through which a manipulator/stabilizer is inserted to be coupled to the retractor to keep the retractor aligned as desired.

It would be desirable to have a reliable, simple to use cardiac retractor for use in minimally invasive surgery which could be deployed through a single minimally invasive incision/opening.

### SUMMARY

The invention is set out in the appended claims.

A cardiac retractor is disclosed. The cardiac retractor has an outer tube. The cardiac retractor also has a fixed collar fixedly coupled to a proximal end of the outer tube. The cardiac retractor further has a fixed link fixedly coupled to a distal end of the outer tube. The cardiac retractor also has an inner tube rotatable within the outer tube. The cardiac retractor further has a fixed key coupled to a proximal end of the inner tube and configured to rotate the inner tube relative to the outer tube. The cardiac retractor also has a keyed link coupled to a distal end of the outer tube.

### BRIEF DESCRIPTION OF THE DRAWINGS

FIG. 1 is a perspective view of one embodiment of a cardiac retractor.
FIGS. 2A-2H and 2J are a series of exploded perspective views which illustrate the partial assembly of the cardiac retractor of FIG. 1. It should be noted that there is intentionally not a FIG. 2I to avoid confusion with the number 21.
FIG. 2K is a schematic elevational view of an embodiment of the deployment cable routing for the assembly of the cardiac retractor of FIG. 1.
FIGS. 2L-2N are a continued series of exploded perspective views which illustrate the remaining assembly of the cardiac retractor of FIG. 1.
FIG. 3A is a top view of the cardiac retractor of FIG. 1 showing cross-section line 3B-3B.
FIG. 3B is a side cross-sectional view of the cardiac retractor of FIG. 3A.
FIG. 4 is a perspective view of one embodiment of a keyhole cannula for use with a cardiac retractor such as the retractor of FIG. 1.
FIGS. 5A, 5B, 5C, 5D, 5E, and 5F are front, left, right, back, top, and bottom views, respectively, of the keyhole cannula of FIG. 4.
FIGS. 6A-6H, 6J-6N, and 6P-6R illustrate an example of a surgical procedure using the cardiac retractor of FIG. 1 and the keyhole cannula of FIG. 4.
FIG. 7 is a perspective view of another embodiment of a cardiac retractor.
FIG. 8A is a top view of the cardiac retractor of FIG. 7 showing cross-section line 8B-8B.
FIG. 8B is a side cross-sectional view of the cardiac retractor of FIG. 8A.
FIGS. 9A and 9B are both partial cross-sectional and partially exposed views which illustrate the deployment cable mechanism for the cardiac retractor embodiment of FIG. 7.
FIG. 10 is a perspective view of a distal end of another embodiment of a cardiac retractor.
FIGS. 11A-11C are schematic elevational views of the distal end of the cardiac retractor of FIG. 10 illustrating various states of deployment of the cardiac retractor.
FIGS. 12A-12B are schematic elevational views of the distal end of another embodiment of a cardiac retractor.
FIGS. 13A-13D are various views of another embodiment of a cardiac retractor device having a cardiac retractor assembly illustrated in the retracted position.
FIG. 14 is a top view of the cardiac retractor assembly of the embodiment of the cardiac retractor device of FIGS. 13A to 13D.
FIGS. 15A and 15B are cross-sectional views of the embodiment of the cardiac retractor assembly taken along section line 15A-15A of Fig. 14.
FIG. 16A and 16B are cross-sectional views of the embodiment of the cardiac retractor assembly taken along section line 16A-16A of Fig. 14.
FIG. 17A and 17B are cross-sectional views of the embodiment of the cardiac retractor assembly taken along section line 17A-17A of Fig. 14.

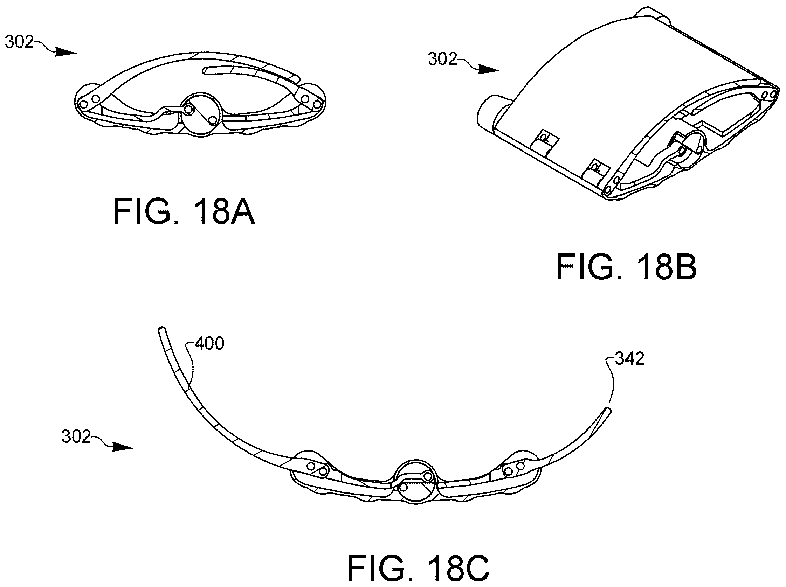

FIG. 18A and 18B are cross-sectional views of the embodiment of the cardiac retractor assembly taken along section line 18A-18A of Fig. 14.
FIG. 18C is a cross-sectional views of the embodiment of the cardiac retractor assembly taken along section line 18A-18A of Fig. 14 when the cardiac retractor assembly is in the extended position.
FIG 19A and 19B are cross-sectional views of the embodiment of the cardiac retractor assembly taken along section line 19A-19A of Fig. 14.
FIG.. 20A is a perspective view of the embodiment of the cardiac retractor assembly when the cardiac retractor assembly is in the extended position.
FIG. 20B is a perspective view of the embodiment of the cardiac retractor assembly when the cardiac retractor assembly is in a first intermediate position.
FIG. 20C is a perspective view of the embodiment of the cardiac retractor assembly when the cardiac retractor assembly is in a second intermediate position.
FIG. 20D is a perspective view of the embodiment of the cardiac retractor assembly when the cardiac retractor assembly is in a third intermediate position.
FIG. 20E is a perspective view of the embodiment of the cardiac retractor assembly when the cardiac retractor assembly is in the retracted position.
FIG. 21A is a perspective view of the embodiment of the cardiac retractor assembly when the cardiac retractor assembly is in the retracted position.
FIG. 21B is a perspective view of the embodiment of the cardiac retractor assembly when the cardiac retractor assembly is in the retracted position and with the second blade omitted for clarity.
FIG. 21C is a perspective view of the embodiment of the cardiac retractor assembly when the cardiac retractor assembly is in the retracted position and with the first blade and the second blade omitted for clarity.
FIG. 22A is rear view of the cardiac retractor device illustrating a transmission assembly with a rear portion of the housing omitted for clarity.
FIG. 22B is rear view of the cardiac retractor device illustrating a transmission assembly with a rear portion of the housing omitted for clarity.
FIG. 23 is a cross-sectional view of the embodiment of the cardiac retractor device taken along section line 23-23 of Fig. 13C.

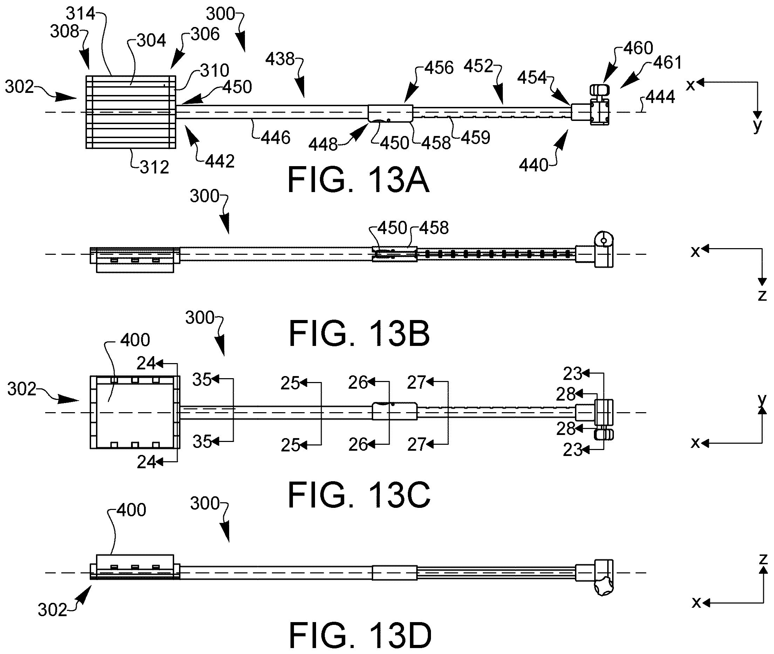

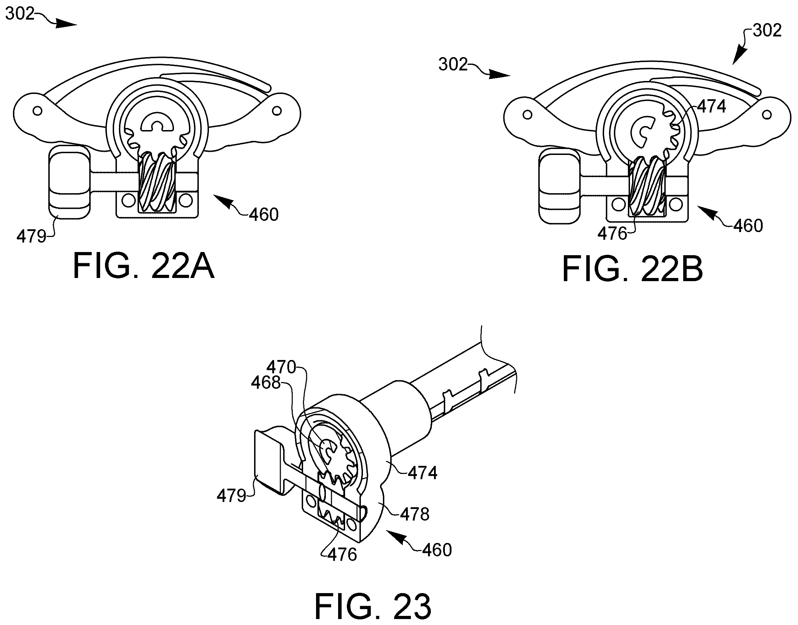

FIG. 23 is a cross-sectional view of the embodiment of the cardiac retractor device taken along section line 23-23 of Fig. 13C.

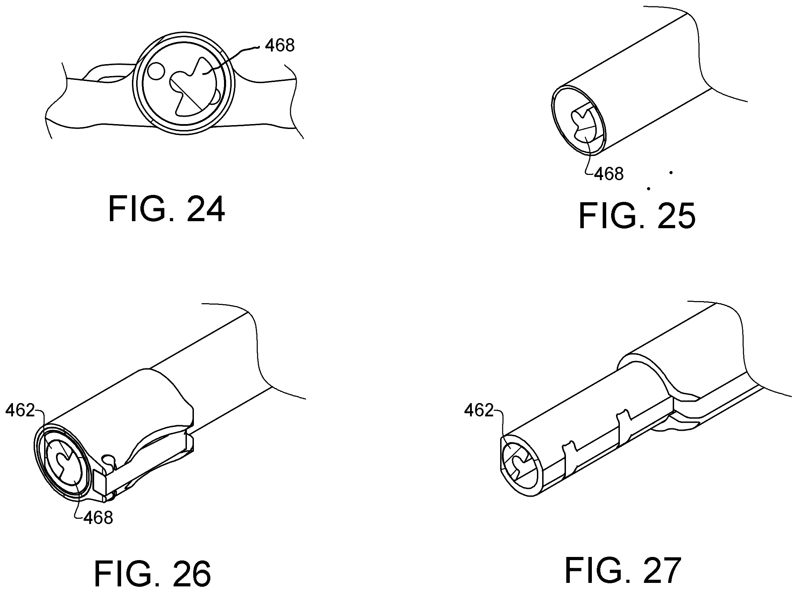

FIG. 24 is a cross-sectional view of the embodiment of the cardiac retractor device taken along section line 24-24 of Fig. 13C.
FIG. 25 is a cross-sectional view of the embodiment of the cardiac retractor device taken along section line 25-25 of Fig. 13C.
FIG. 26 is a cross-sectional view of the embodiment of the cardiac retractor device taken along section line 26-26 of Fig. 13C.
FIG. 27 is a cross-sectional view of the embodiment of the cardiac retractor device taken along section line 27-27 of Fig. 13C.

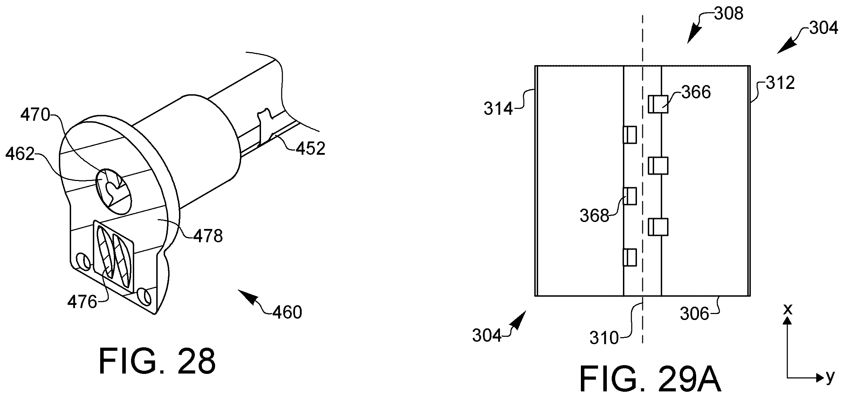

FIG. 28 is a cross-sectional view of the embodiment of the cardiac retractor device taken along section line 28-28 of Fig. 13C.

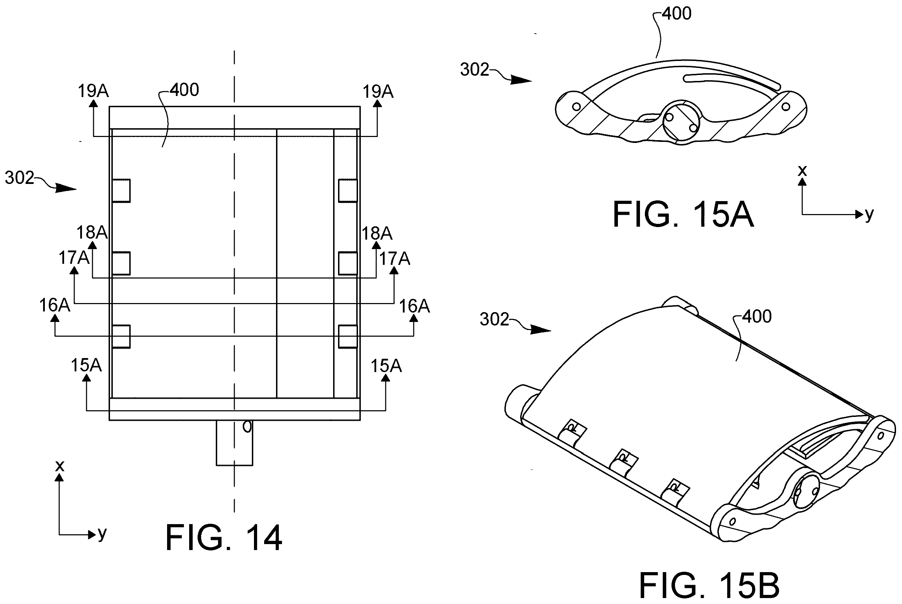

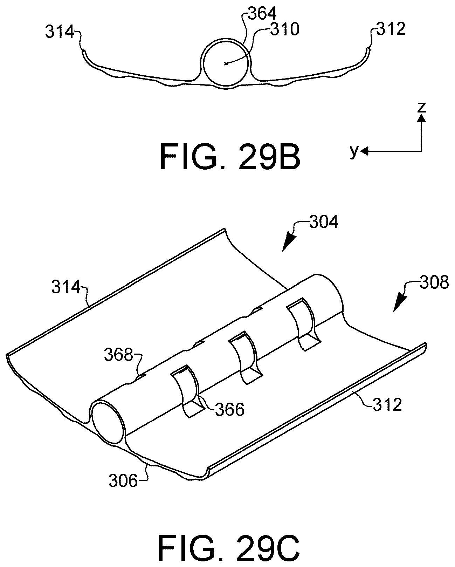

FIGS. 29A to 29C are various views of a base plate of the embodiment of the cardiac retractor assembly of FIG. 14.

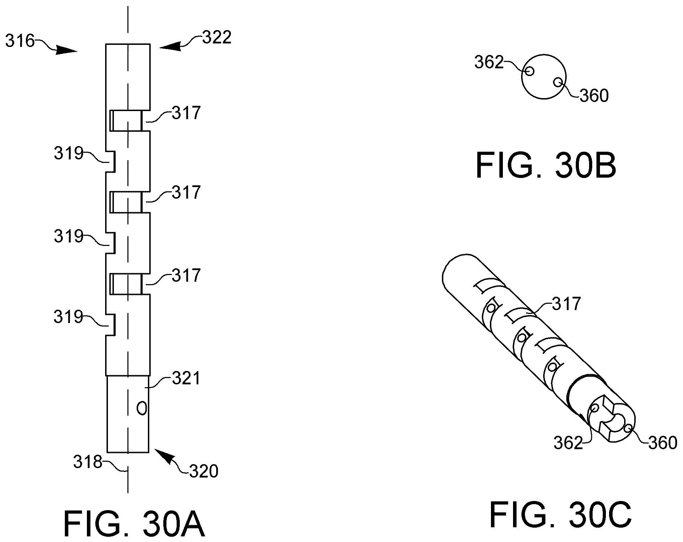

FIGS. 30A to 30E are various views of a hub of the embodiment of the cardiac retractor assembly of FIG. 14.

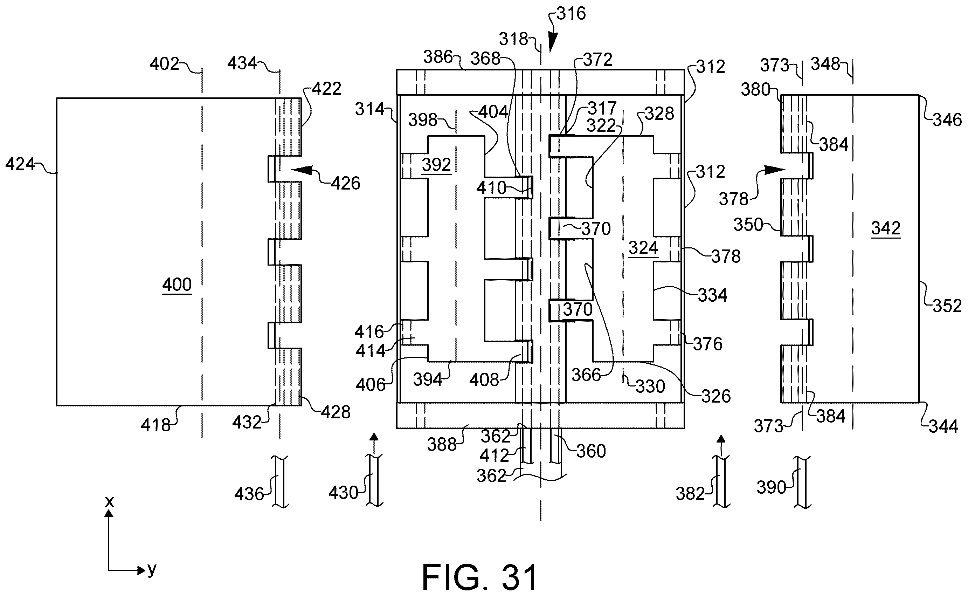

FIG. 31 is a top exploded view of the cardiac retractor assembly of FIG. 14.

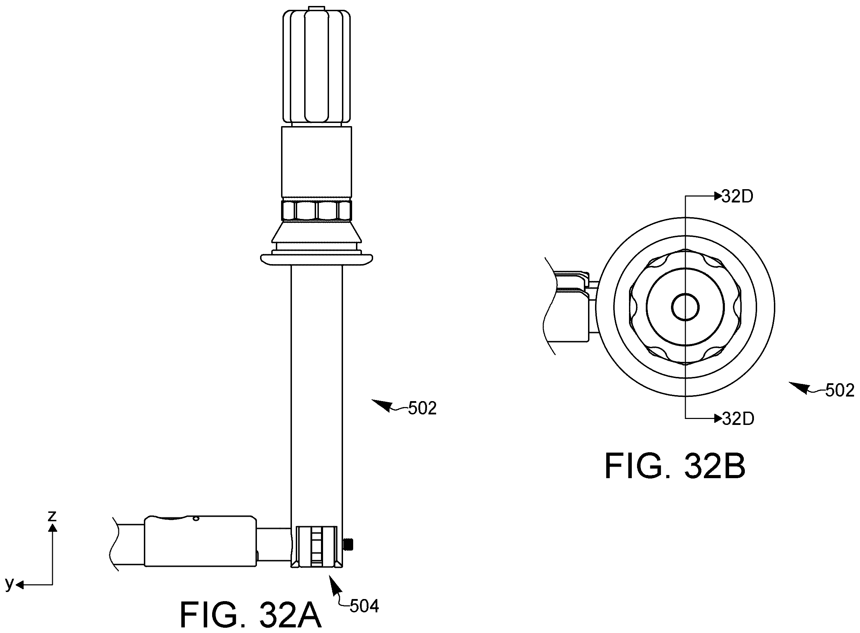

FIG. 32A is a perspective views of another embodiment of a cardiac retractor device having a cardiac retractor assembly illustrated in the retracted position.

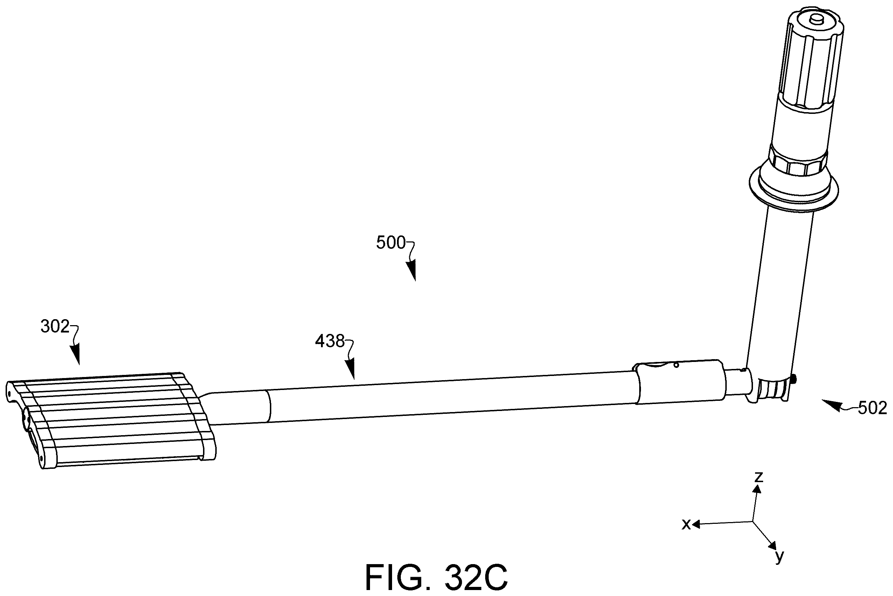

FIGS. 32B and 32C are side and top views, respectively, of an actuation assembly of the cardiac retractor device of FIG. 32A.

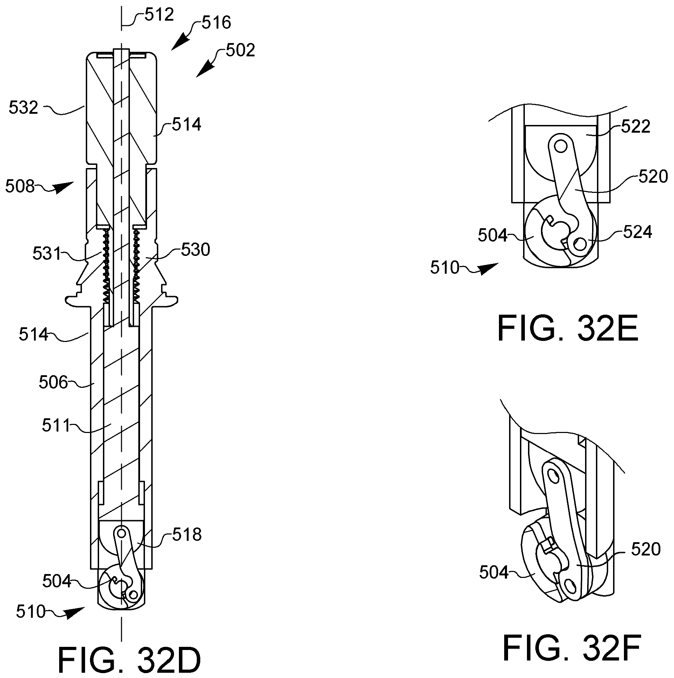

FIG. 32D is a cross-sectional view of the embodiment of the actuation assembly taken along section line 32D-32D of Fig. 32B.
FIG. 32E and 32F is a detail of the distal end of the cross-sectional view of the embodiment of the actuation assembly of Fig. 32D.

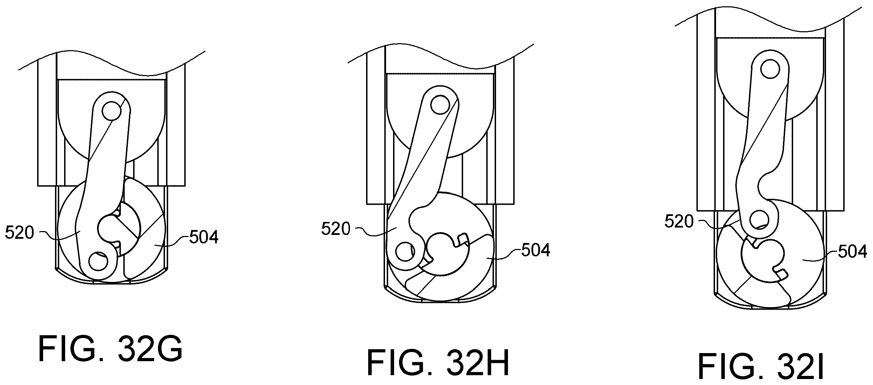

FIGS. 32G to 32I are a detail of the distal end of the cross-sectional view of the embodiment of the actuation assembly of Fig. 32D with a collar in various rotational positions.

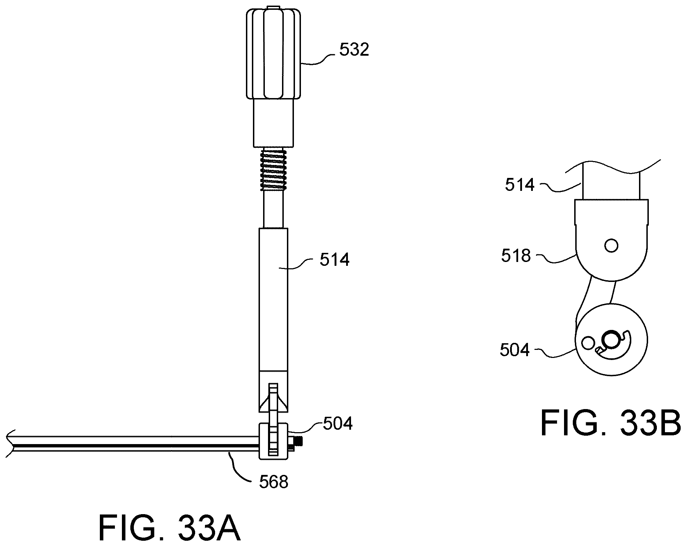

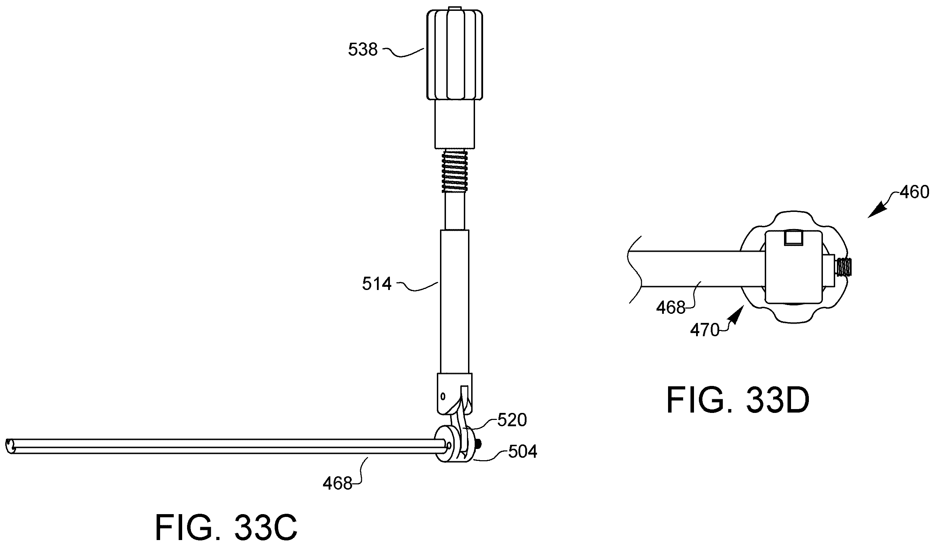

FIGS. 33A to 33D are various views of the of the actuation assembly of FIG. 32A with the housing and a portion of the shaft portion omitted for clarity.

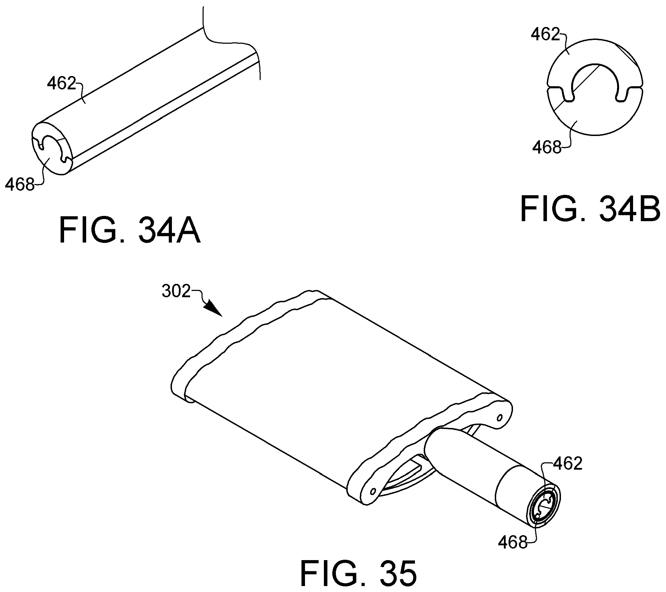

FIGs 34A and 34B are a detailed view of a cross-section of the first transmission rod and the second transmission rod with a portion of the shaft portion omitted for clarity.
FIG 35 is a perspective cross-sectional view of the embodiment of the cardiac retractor device taken along section line 35-35 of Fig. 13C.

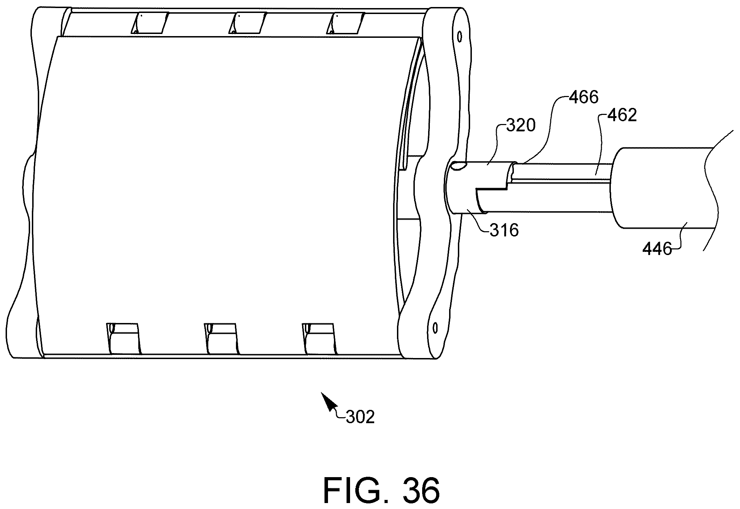

FIG. 36 is a perspective cross-sectional view of the cardiac retractor assembly of FIG. 13A with a portion of the housing portion omitted for clarity.

It will be appreciated that for purposes of clarity and where deemed appropriate, reference numerals have been repeated in the figures to indicate corresponding features, and that the various elements in the drawings have not necessarily been drawn to scale in order to better show the features.

### DETAILED DESCRIPTION

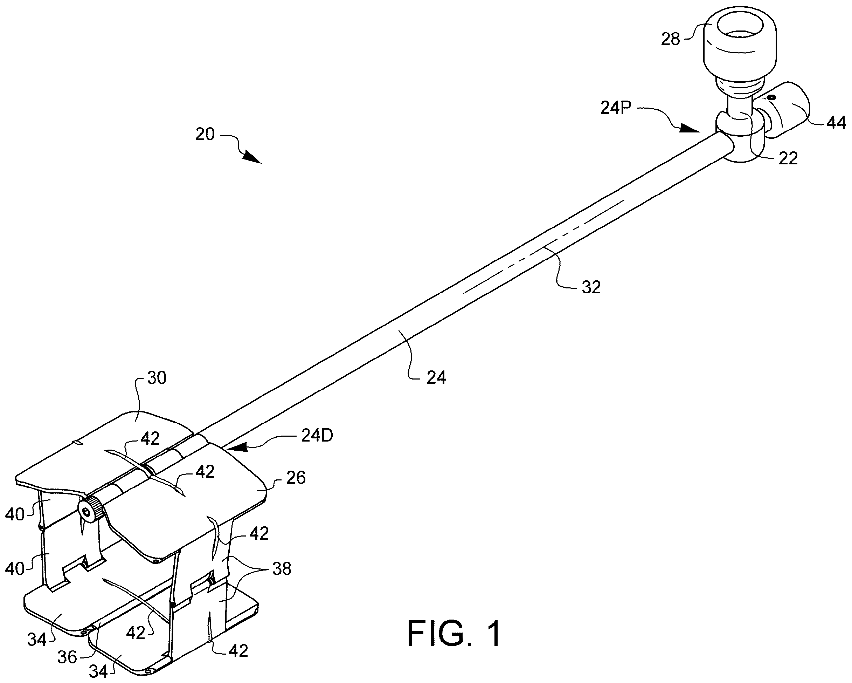

FIG. 1 illustrates one embodiment of a cardiac retractor 20. The retractor has a fixed collar 22 which is fixedly attached to the proximal end 24P of an outer tube 24. A fixed link 26 is fixedly attached to the distal end 24D of the outer tube 24. A coaxial nut 28 is coupled to a keyed link 30 by elements which are not visible in this view, but which will be shown and discussed later in this specification. The coaxial nut 28 is coupled to a fixed key (not visible in this view) which can be rotated around an axis 32 of the outer tube 24 to cause a related movement of the keyed link 30. As shown in FIG. 1, the with the coaxial nut 28 aligned over the fixed collar 22, the keyed link 30 is opened or deployed relative to the fixed link 26.

In this embodiment, the cardiac retractor 20 has a series of additional links in addition to the fixed link 26 and the keyed link 30. Two opposing links 34 are located opposite the keyed link 30 and the fixed link 26. The opposing links 34 are coupled together by a coupling link 36. A first opposing link 34 is coupled to the fixed link 26 by two side links 38, while a second opposing link 34 is coupled to the keyed link 30 by two side links 40. Each link in a pair of adjacent links (30/40, 40/40, 40/34, 34/36, 36/34, 34/38, 38/26) is pivotable relative to each other. A deployment cable 42 is routed around the links 26, 38, 38, 34, 36, 34, 40, 40, 30 in a pattern which will cause the links to open into the deployed state shown in FIG. 1 when tension is applied to the deployment cable 42 by a cable tension screw knob 44.

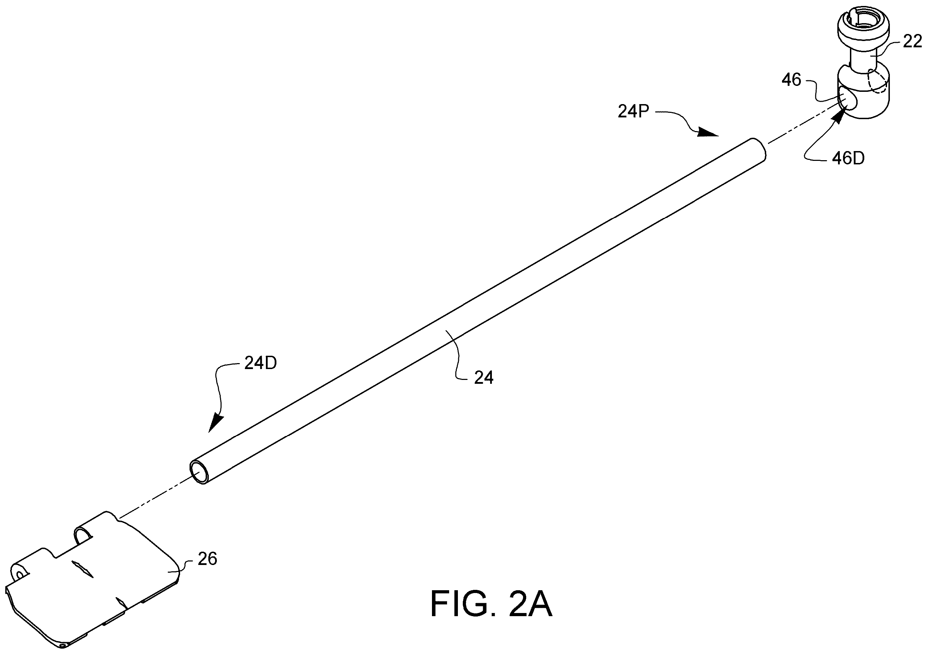

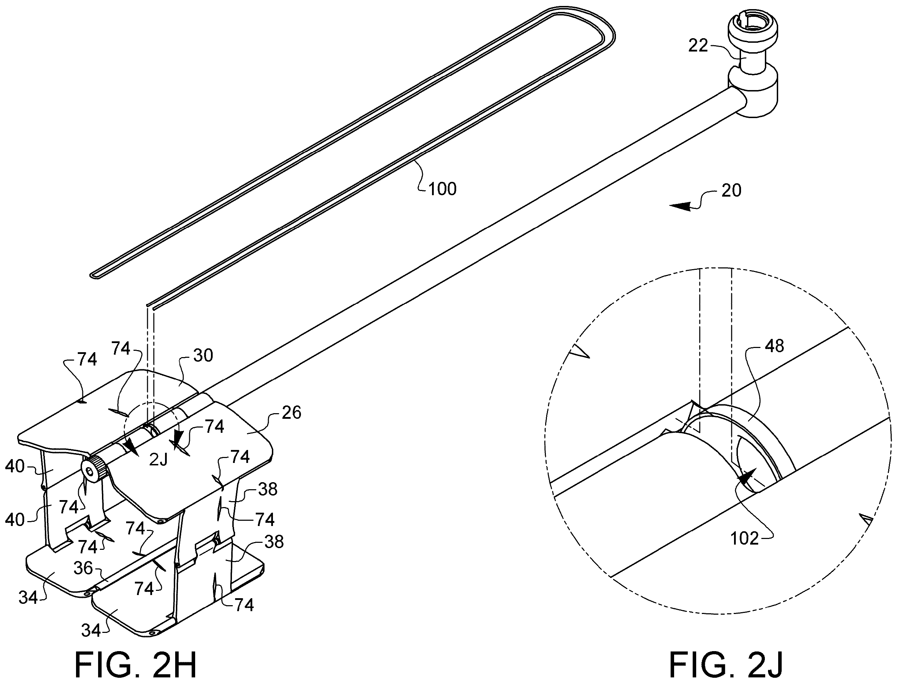

FIGS. 2A-2H and 2J are a series of exploded perspective views which illustrate the partial assembly of the cardiac retractor of FIG. 1. It should be noted that there is intentionally not a FIG. 2I to avoid confusion with the number 21. As shown in FIG. 2A, the outer tube 24 is coupled to the fixed link 26 at the distal end 24D of the outer tube 24. The proximal end 24P of the outer tube 24 is coupled to the fixed collar at a distal side 46D of a passthrough hole 46 in the fixed collar 22.

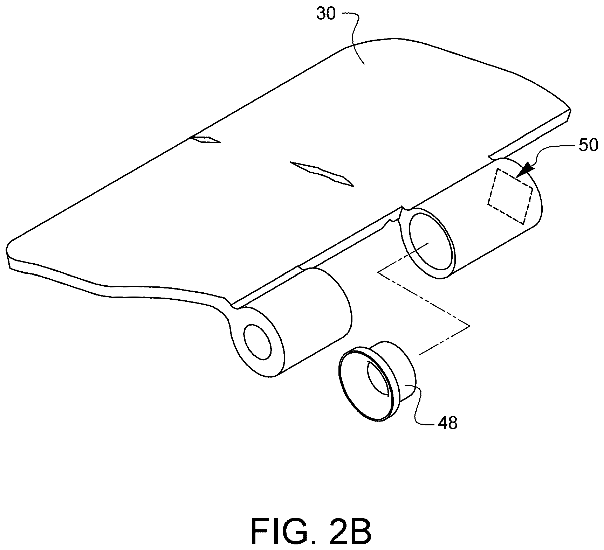

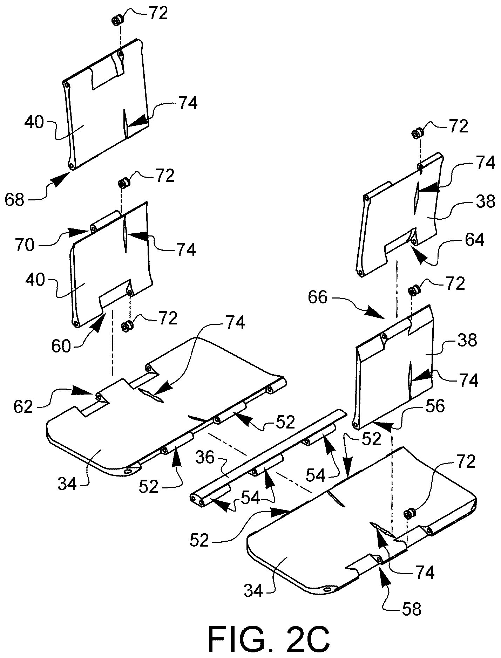

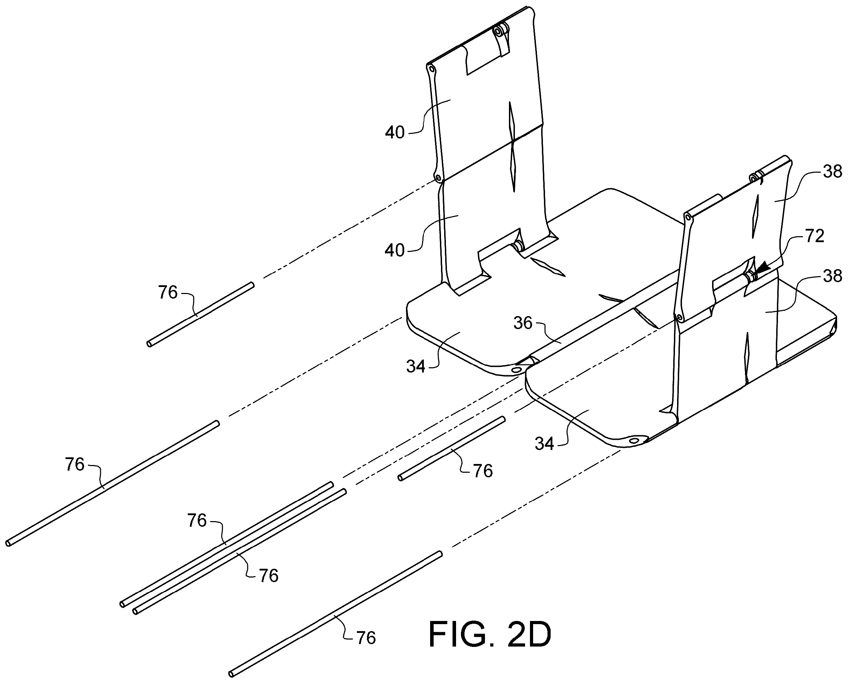

As shown in FIG. 2B, a cable bushing 48 is coupled to the keyed link 30. The keyed link 30 has a keyed opening 50 to which reference will be made in a later figure. As shown in FIG. 2C, hinges 52 of the opposing links 34 are aligned with corresponding hinges 54 of the coupling link 36. Similarly, hinge 56 of one of the side links 38 is aligned with corresponding hinge 58 of the first opposing link 34, while hinge 60 of one of the side links 40 is aligned with corresponding hinge 62 of the second opposing link 34. Also similarly, hinge 64 of another of the side links 38 is aligned with hinge 66 of the other side link 38, while hinge 68 of another of the side links 40 is aligned with the hinge 70 of the other side link 40. A series of side rollers 72 is aligned with a hinge on each of the links 38, 40, 34. The side rollers 72 line up with cable guide slots 74 in the links. As shown in FIG. 2D, a series of hinge pins 76 is used to hold each of the hinged joints between links 38, 40, 34 together, including the side rollers 72.

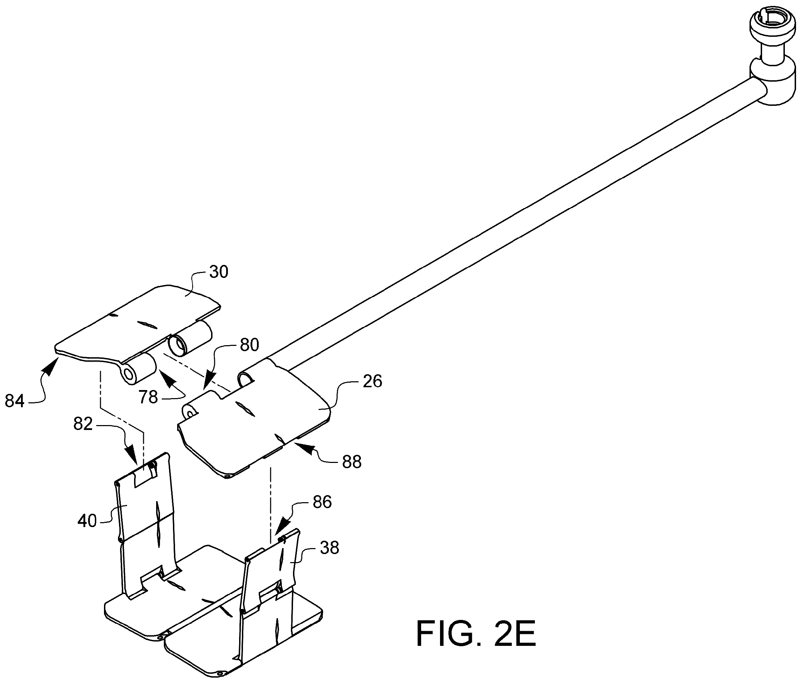

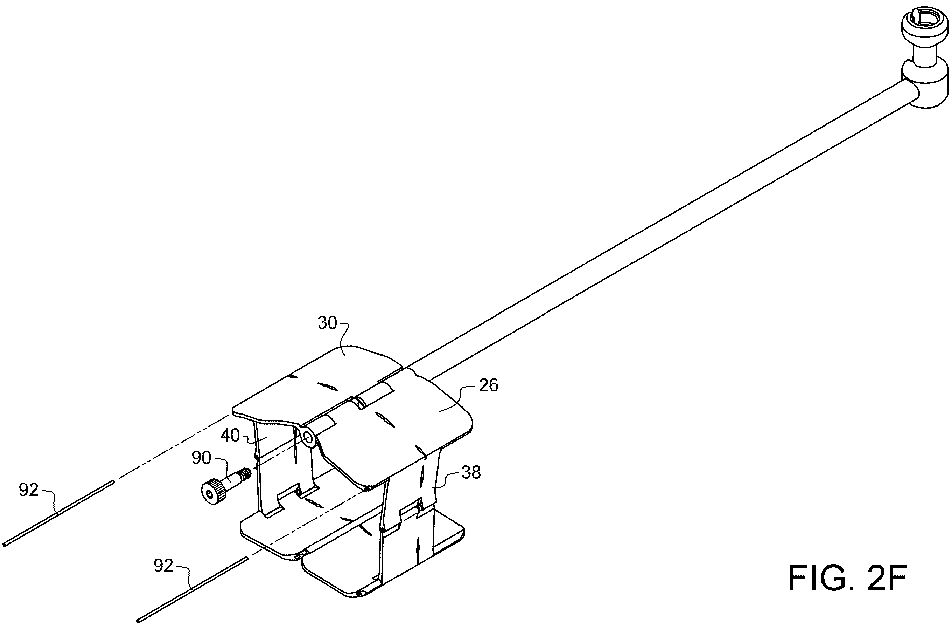

As shown in FIG. 2E, a hinge 78 of the keyed link 30 is brought into alignment with a hinge 80 of the fixed link 26. A hinge 82 of the side link 40 on one end is aligned with a hinge 84 of the keyed link 30. Similarly, a hinge 86 of the side link 38 on another end is aligned with a hinge 88 of the fixed link 26. As shown in FIG. 2F, the keyed link 30 is held pivotably coupled to the fixed link 26 by a threaded axle 90 which passed through the end of the keyed link 30 and screws into the fixed link 26. Hinge pins 92 are used to pivotably couple the keyed link 30 and fixed link 26 to their respective side links 40, 38.

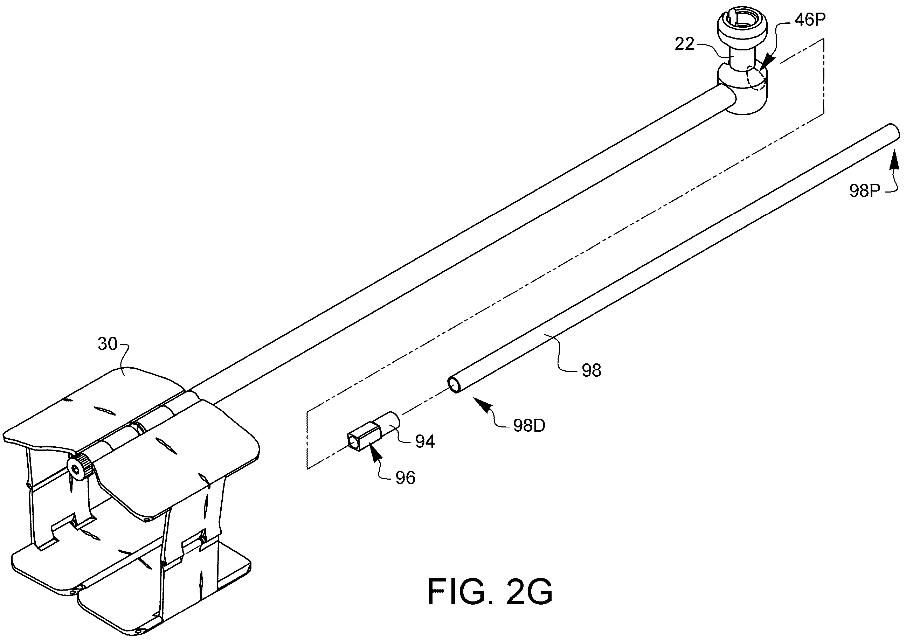

As shown in FIG. 2G, a tube drive 94 having a keyed end 96 is coupled to the distal end 98D of an inner tube 98. The assembled tube drive 94 and inner tube 98 are then inserted into the proximal side opening 46P of a passthrough hole 46 in the fixed collar 22 until the keyed end 96 of the tube drive 94 engages the keyed opening 50 (visible in FIG. 2B) of the keyed link 30.

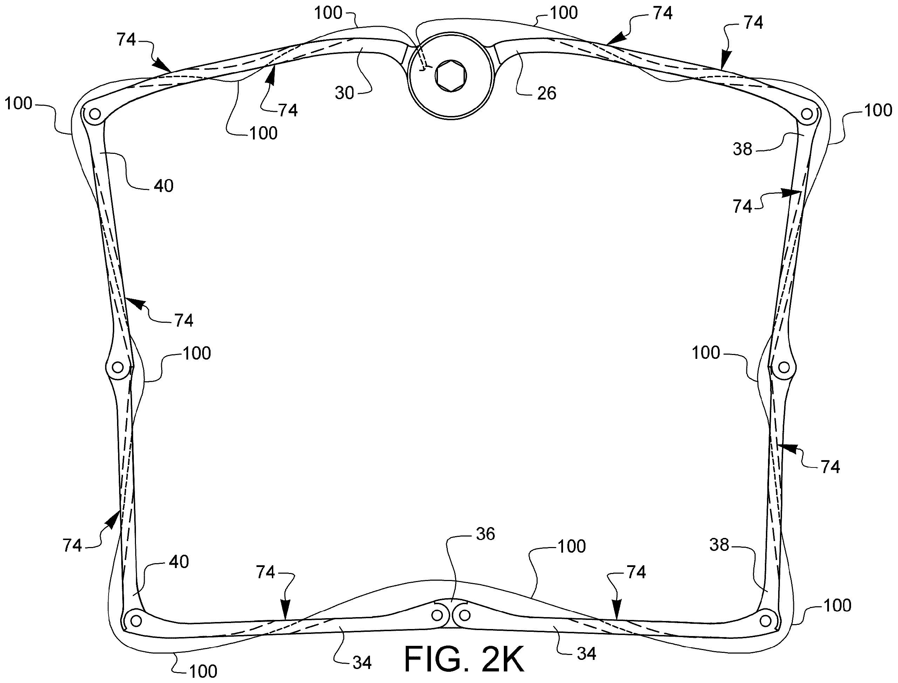

As shown in FIG. 2H, a deployment cable 100 is added to the cardiac retractor 20. The deployment cable 100 is routed around the links 26, 38, 38, 34, 36, 34, 40, 40, 30 as shown in the side view of FIG. 2K. FIG. 2J is an enlarged view of a portion of FIG. 2H, which shows an opening 102 in the cable bushing 48. After routing the deployment cable 100 around the links as shown in FIG. 2K, the ends of the deployment cable 100 are passed through the opening 102 in the cable bushing 48, passed down through the inner tube 98 (previously visible in FIG. 2G), and out of the opening 46P in the fixed collar 22 (also previously visible in FIG. 2G).

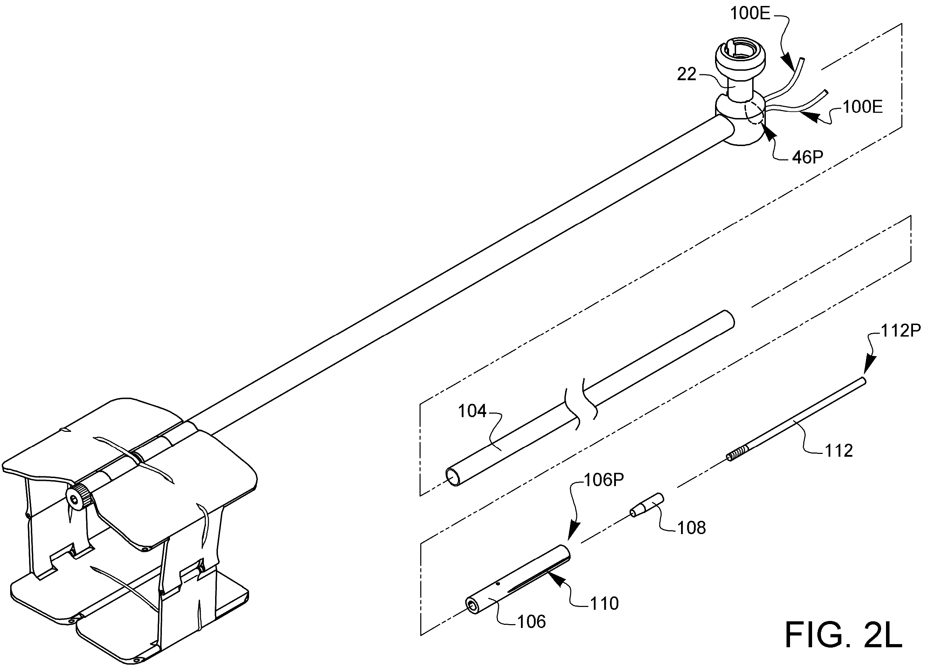

The ends 100E if the deployment cable 100 are shown exiting the opening 46P in FIG. 2L. An assembly spacer 104 (also known as a cable tube) is placed over the deployment cable ends 100E and into the opening 46P and slide inside the inner tube 98 (previously visible in FIG. 2G). A tension nut 106 is also placed over the deployment cable ends 100E and into the opening 46P to slide against the assembly spacer 104. A cable lock wedge 108 is pressed into the proximal end 106P of the tension nut 106, pinning the deployment cable 100 within the tension nut 106 while forcing the deployment cable ends 100E into slots 110 in the tension nut 106. Excess cable ends 100E are then trimmed at the tension nut 106. A threaded rod 112 is then threaded into the tension nut 106.

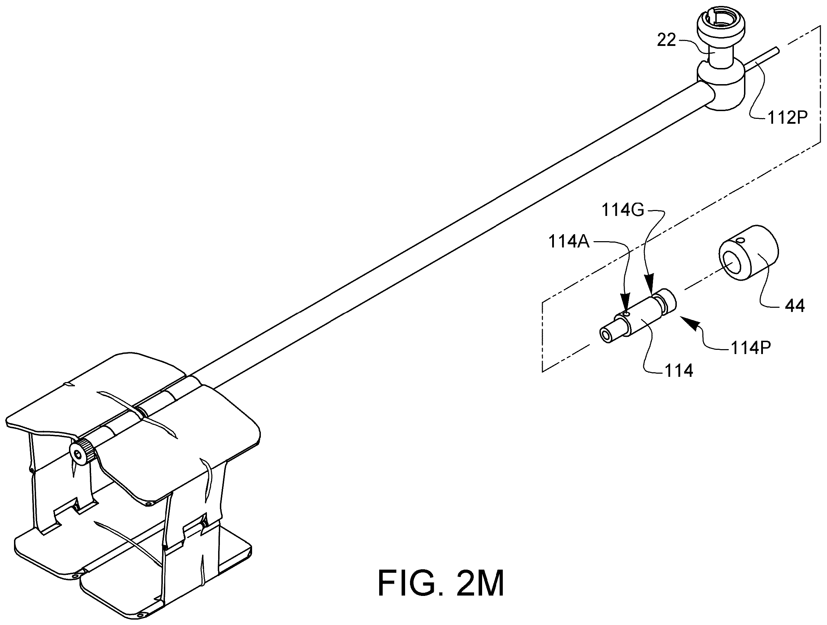

As shown in FIG. 2M, the proximal end 112P of threaded rod 112 protrudes from the fixed collar 22. A key mount collar 114 is passed over the proximal end 112P of threaded rod 112 and coupled to the inner tube 98 (previously visible in FIG. 2G). In the view of FIG. 2M, we also call out the attachment hole 114A and the groove 114G of key mount collar 114, since both will be referred to in the following figure. The cable tension screw knob 44 is then placed over the proximal end 114P of the key mount collar 114 while also being fixedly coupled to the proximal end 112P of threaded rod 112.

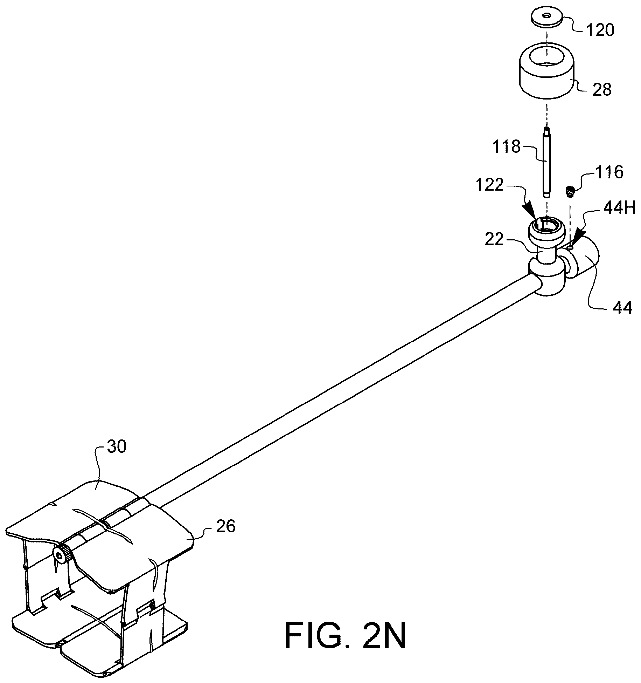

As shown in FIG. 2N, a set screw 116 is threaded into hole 44H of the cable tension screw knob 44 so that the set screw 116 also rides in the groove 114G of the key mount collar 114 (previously visible in FIG. 2M). A fixed key 118 is passed through the fixed collar 22 and coupled to the attachment hole 114A of the key mount collar 114. The coaxial nut 28 is slid over the fixed key 118, while a retaining washer 120 is coupled to the end of the fixed key 118 in order to keep the coaxial nut 28 from coming off the fixed key 118. The fixed collar 22 has a slot 122 which is sized to allow the fixed key 118 to be rotated out of the fixed collar 22 when the coaxial nut 28 is not threaded onto the fixed collar 22. Rotating the fixed key 118, rotates the key mount collar 114 to which it is connected, which in turn rotates the inner tube 98 (previously visible in FIG. 2G) to which it is connected, which in turn rotates the tube drive 94, which in turn rotates the keyed link 30 to which it is connected. When the fixed key 118 is aligned with the fixed collar 22, the keyed link 30 is in the open or deployed position shown in FIG, 2N. When the fixed key 118 is rotated out of the fixed collar 22, the keyed link 30 pivots towards the fixed link 26 for a closed or undeployed position (not shown in this view).

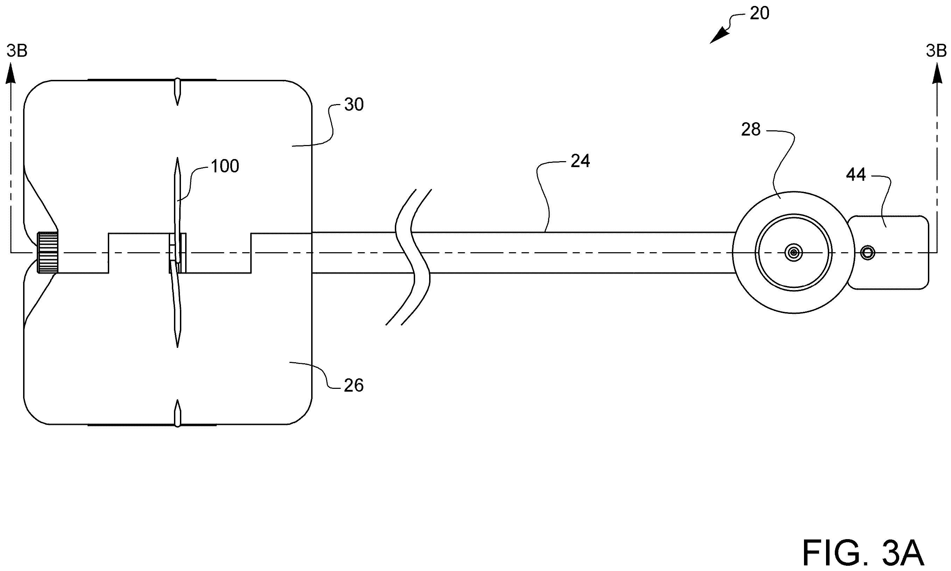

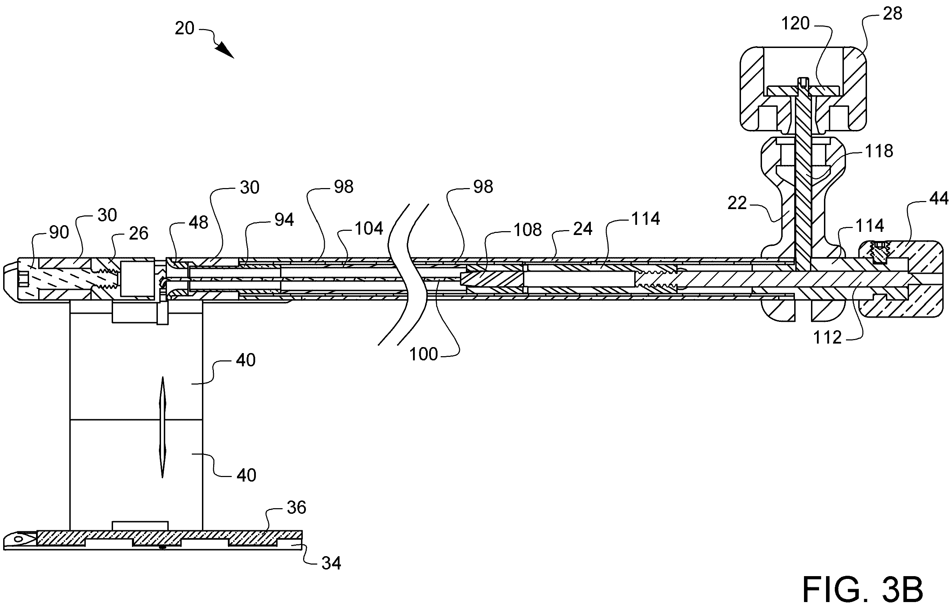

FIG. 3A is a top view of the cardiac retractor 20 of FIG. 1 showing cross-section line 3B-3B. FIG. 3B is a side cross-sectional view of the cardiac retractor 20 of FIG. 3A.

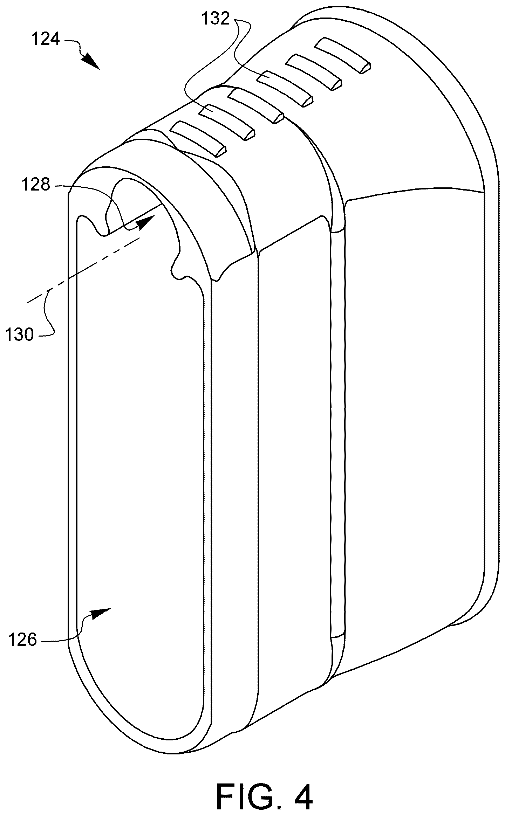

FIG. 4 is a distal perspective view of one embodiment of a keyhole cannula 124 for use with a cardiac retractor 20 such as the retractor of FIG. 1. The keyhole cannula 124 has an instrument passage 126 which in this embodiment is oblong in shape. Coupled to the instrument passage 126 is a key hole 128 which is sized to accept the outer tube 24 of the cardiac retractor 20 such that the device may only be slid into the key hole 128 along a key hole axis 130 which is substantially perpendicular to the opening of the passage 126. To accomplish this, the key hole 128 covers an arc of more than 180 degrees, thereby preventing a tube large enough to fill the whole key hole 128 from leaving the key hole 128 unless withdrawn axially. In other embodiments, the key hole axis might not be substantially perpendicular to the opening of the passage, however, the fixed alignment between the key hole axis 130 and the cannula 124 means that the cannula will always be facing a known direction relative to an instrument being placed into the key hole 128, thereby helping to ensure that the cannula is aligned with the instrument being placed therein and engaging the key hole 128. This can help facilitate visualization for the surgeon through the cannula to the point where work is being done inside the patient. In this embodiment, the cannula 124 also has some ribbing 132 and is sized to fit within an intercostal space of a patient's rib cage.

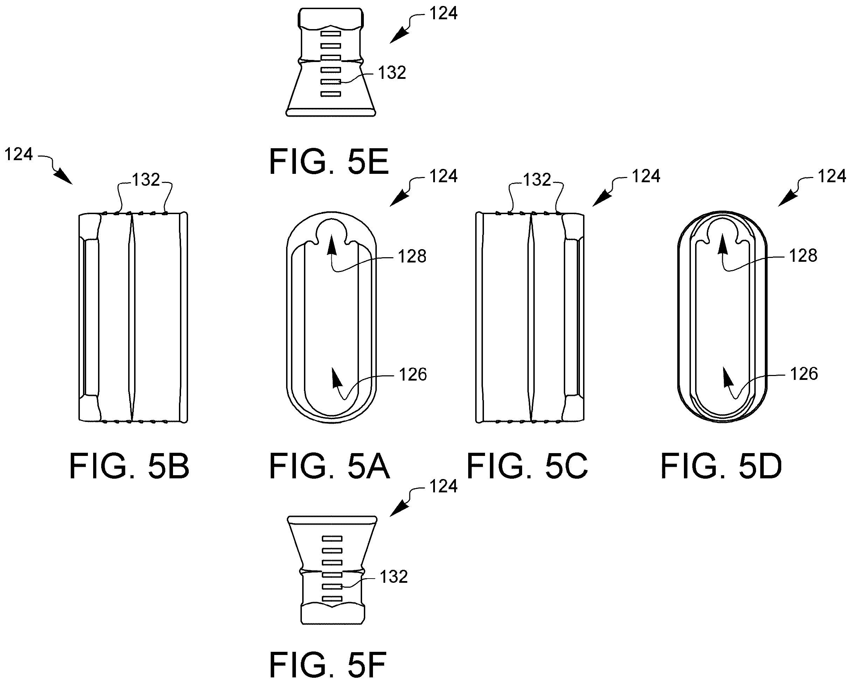

FIGS. 5A, 5B, 5C, 5D, 5E, and 5F are front, left, right, back, top, and bottom views, respectively, of the keyhole cannula 124 of FIG. 4.

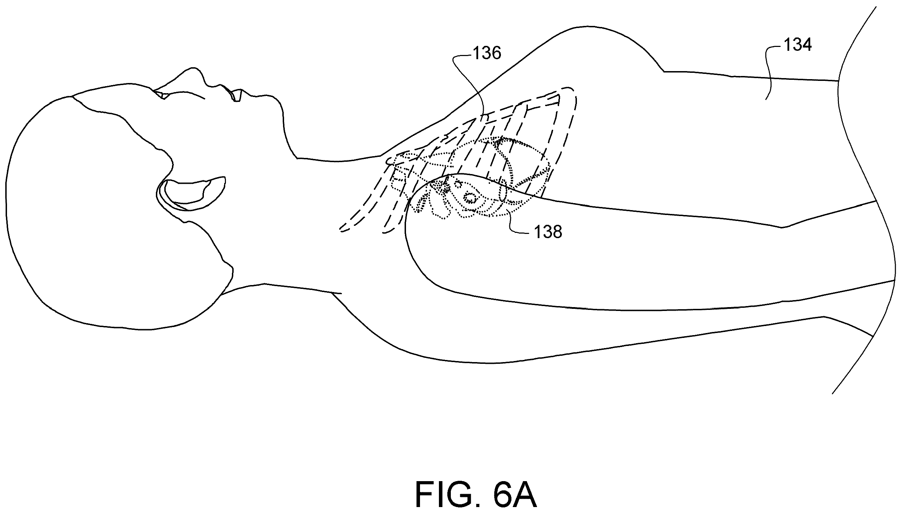

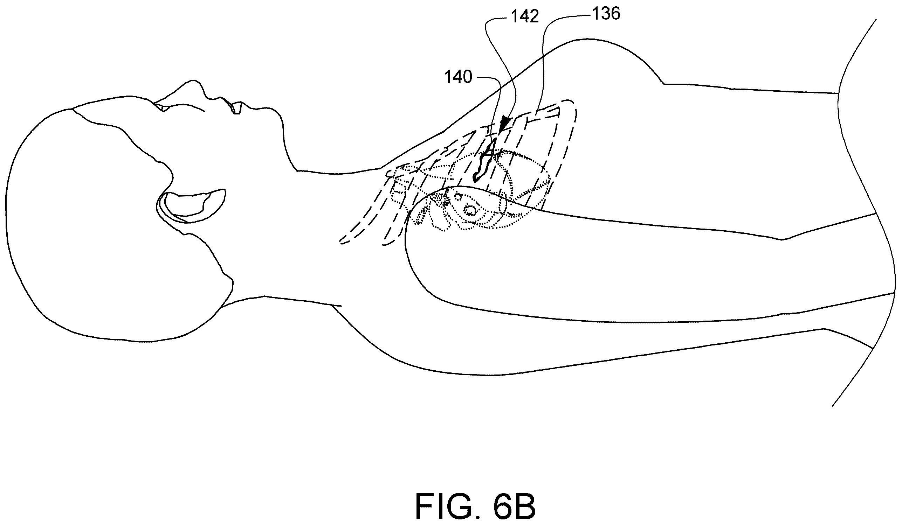

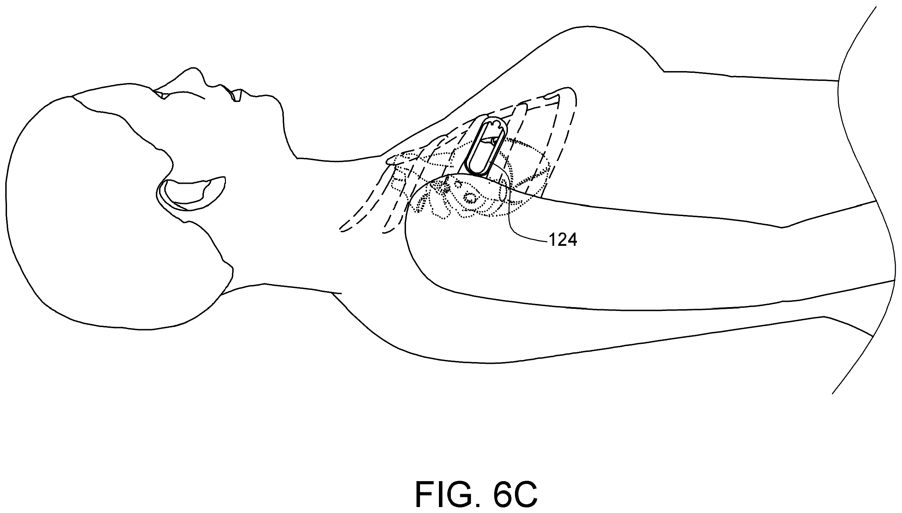

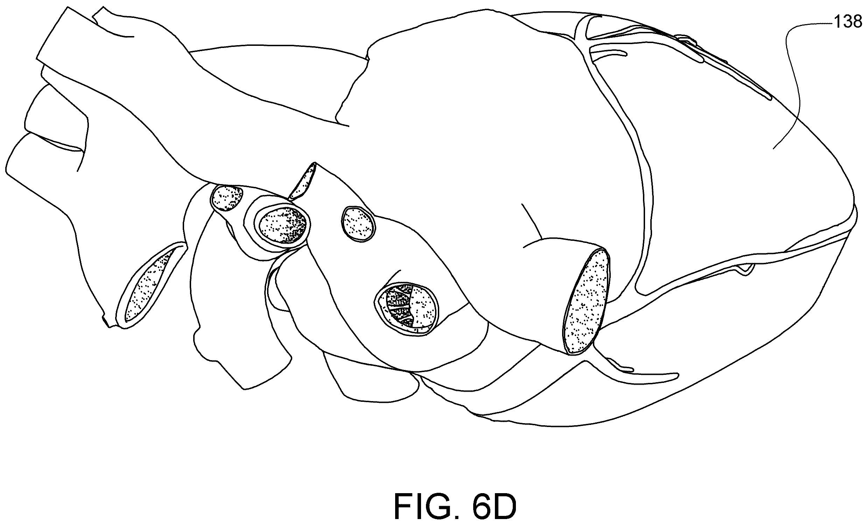

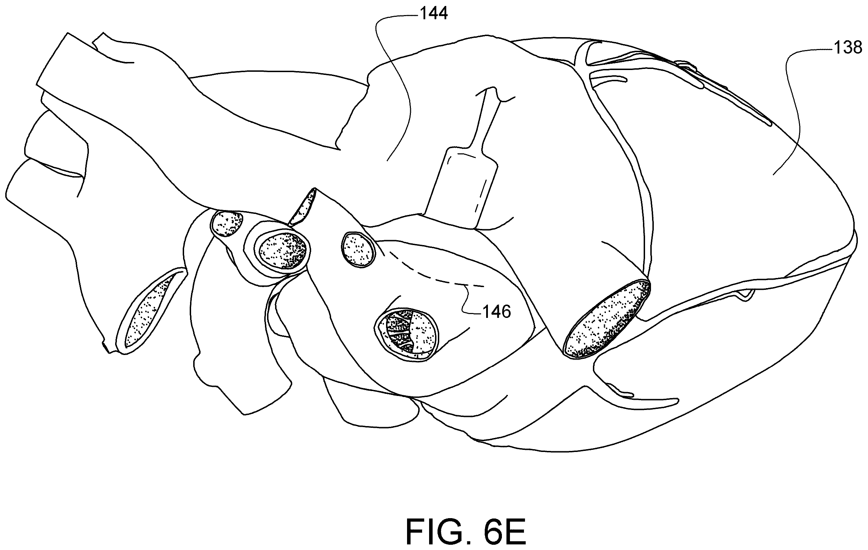

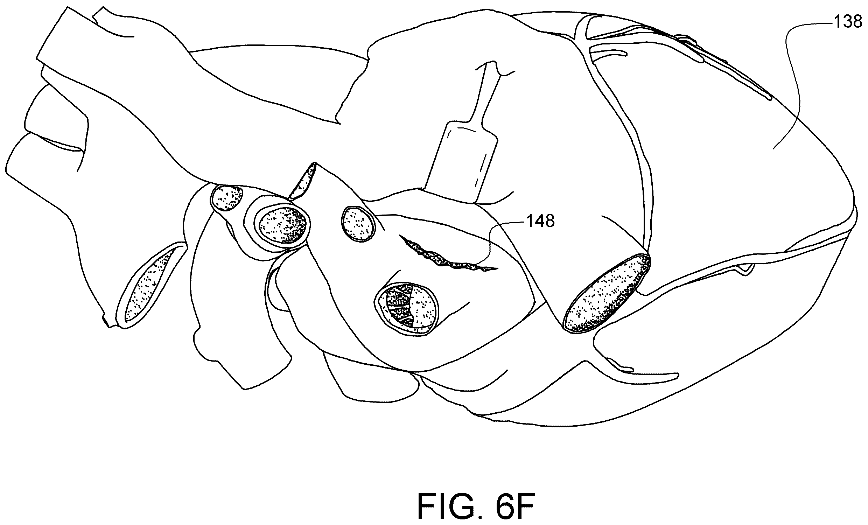

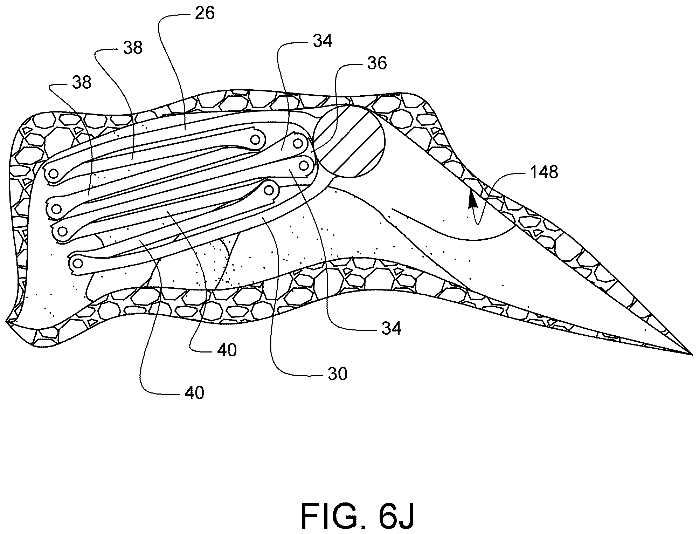

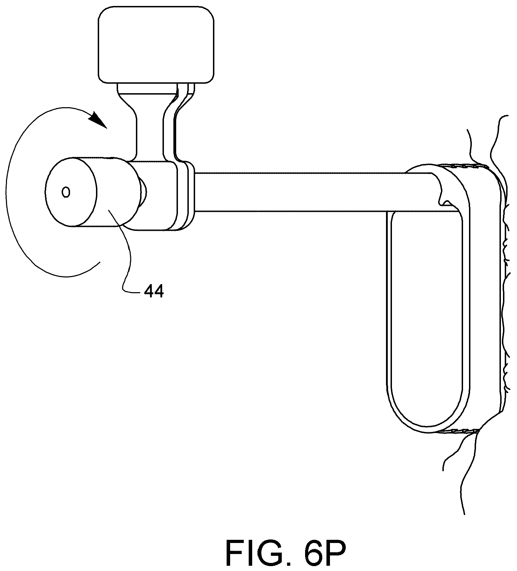

FIGS. 6A-6H, 6J-6N, and 6P-6R illustrate an example of a surgical procedure using the cardiac retractor of FIG. 1 and the keyhole cannula of FIG. 4. FIG. 6A illustrates a patient 134 with a portion of their ribcage 136 visible, and their heart 138 within the thoracic cavity. As shown in FIG. 6B, an incision 140 is made in an intercostal space 142 of the ribcage 136. As shown in FIG. 6C, a keyhole cannula 124 may be placed within the intercostal incision 140. The long outer sides of the cannula 124 are smooth and do not have ribbing in order to avoid traumatizing nerves and vessels which may run along the adjacent ribs. FIG. 6D is an enlarged schematic view of the heart 138, such as a surgeon might see when looking through the cannula 124. As shown in FIG. 6E, vessels 144 may be manipulated to locate a desired incision point 146 on the wall of the heart 138. As shown in FIG. 6F, an incision 148 may be made in the wall of the heart 138 at the desired location. In this example, the incision 148 has been made into the left atrium of the heart 138.

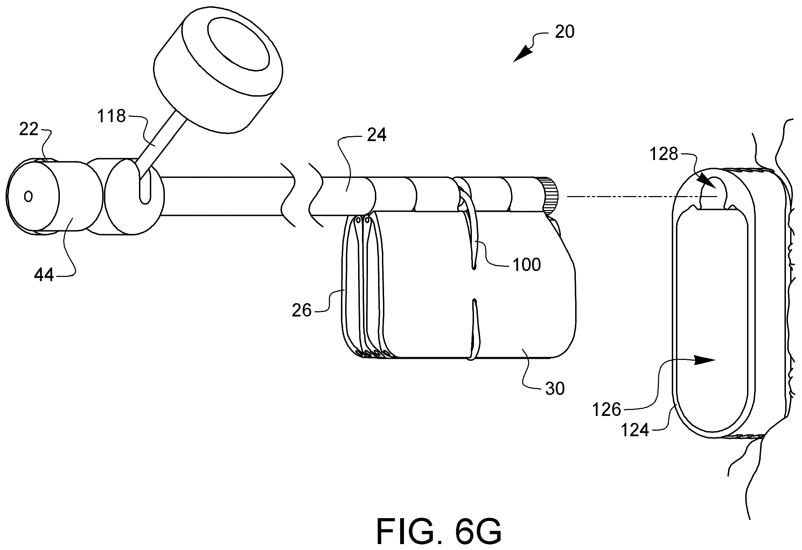

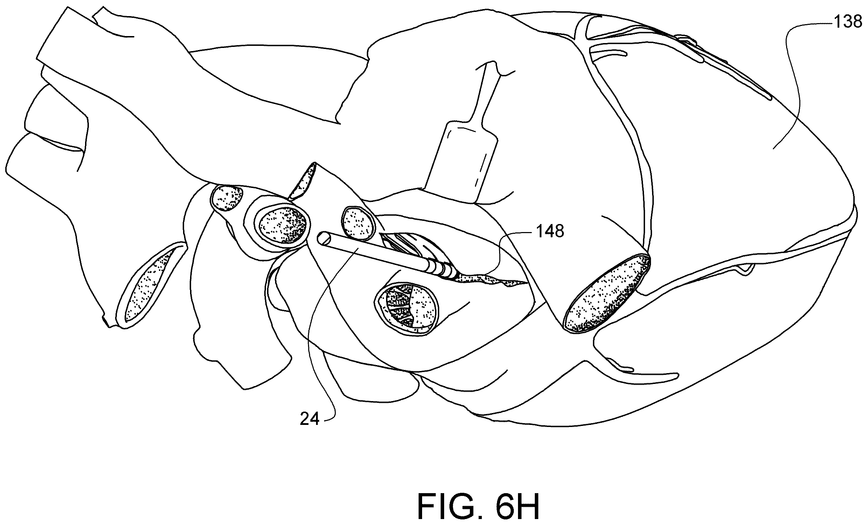

FIG. 6G refocuses on the outside of the patient. The cardiac retractor 20 having its fixed key 118 is rotated out of the fixed collar 22 so that the keyed link 30 is rotated down towards the fixed link 26, and with the other links folded therebetween because tension has not been applied to the deployment cable 100 by the tension knob 44 is inserted into the key hole 128 of the keyhole cannula 124. The folded links 26, 30 fit within the cannula passage 126, while the key hole 128 is sized to hold the outer tube 24. Once the links have passed through the cannula, the outer tube 24 can be rotated within the cannula 124 to orient the folded links as desired. As shown in FIG. 6H, looking back at the heart 138 again, the folded links have been rotated to align with the incision 148. This is shown in a schematic elevational view of FIG. 6J, where the folded links 26, 38, 38, 34, 36, 34, 40, 40, and 30 can be seen in a closed or undeployed configuration. For simplicity, the deployment cable is not shown in this view.

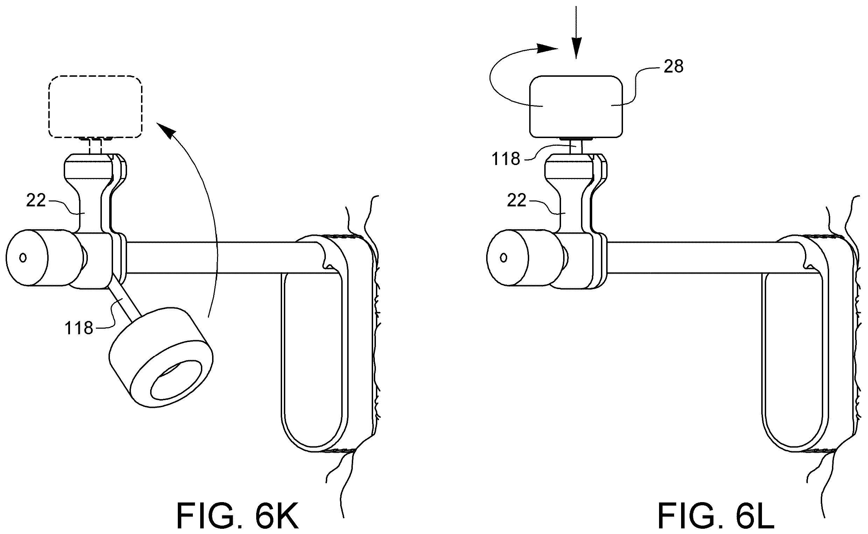

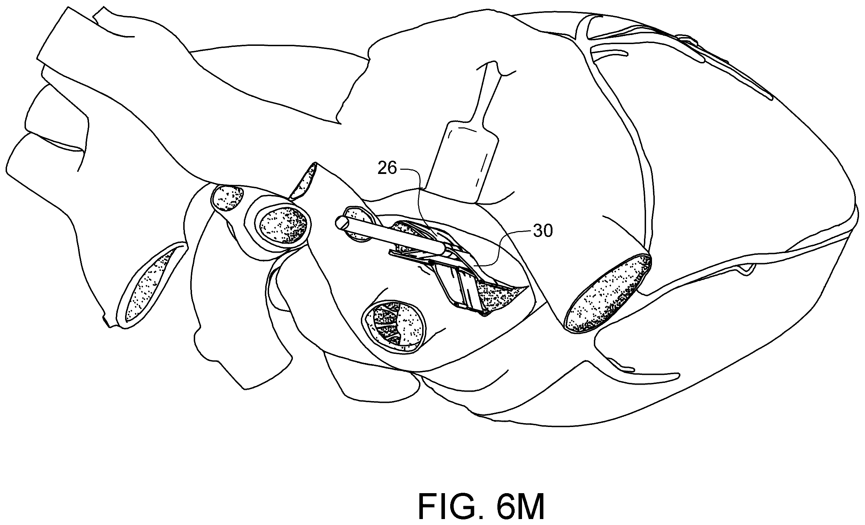

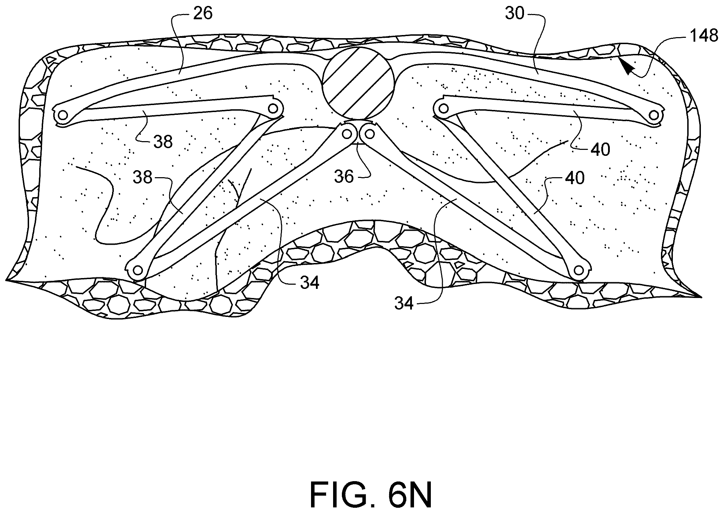

As shown in FIG. 6K, outside the patient again, the fixed key 118 is then rotated into alignment with the fixed collar 22, and then, as shown in FIG. 6L, the coaxial nut 28 is threaded into onto threads within the fixed collar 22 to hold the fixed key 118 in this deployed position. Inside the patient, this rotation of the fixed key 118 into alignment with the fixed collar 22 results in the keyed link 30 pivoting to the open/deployed position relative to the fixed link 26 as shown in the perspective view of FIG. 6M and the schematic elevational view of FIG. 6N.

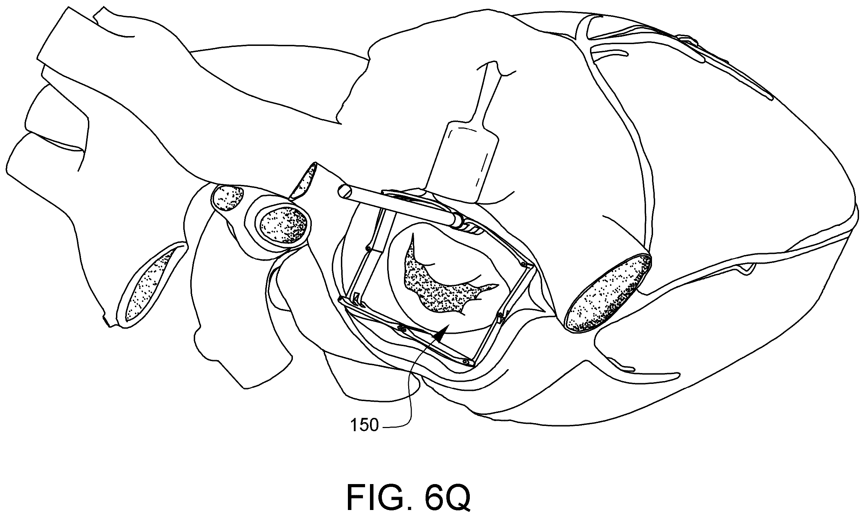

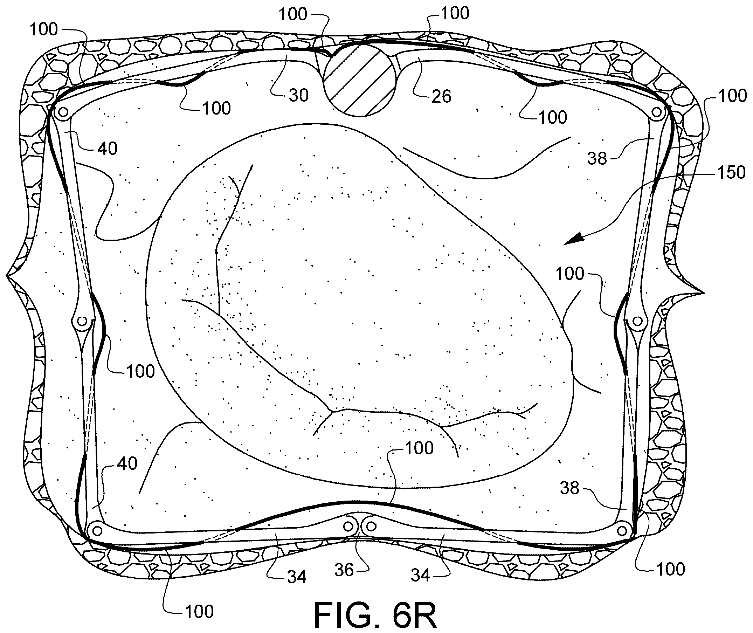

FIG. 6P refocuses outside the patient again. The tension knob 44 is tightened, drawing the tension nut 106 proximally towards the user, and applying tension to the deployment cable 100. This causes the links 38, 38, 34, 36, 36, 40, and 40 to create an opening 150, visible in the inside-patient-views of FIGS. 6Q and 6R, which effectively retracts the wall of the heart, allowing for surgical access. For simplicity, the deployment suture is not shown in FIG. 6Q, however, the tensioned deployment cable is shown schematically in FIG. 6R.

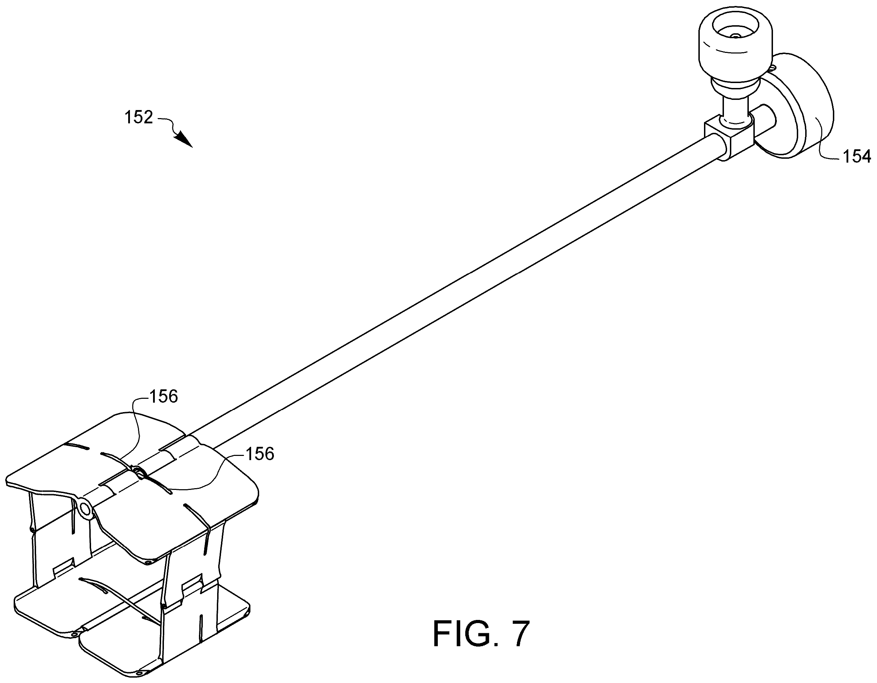

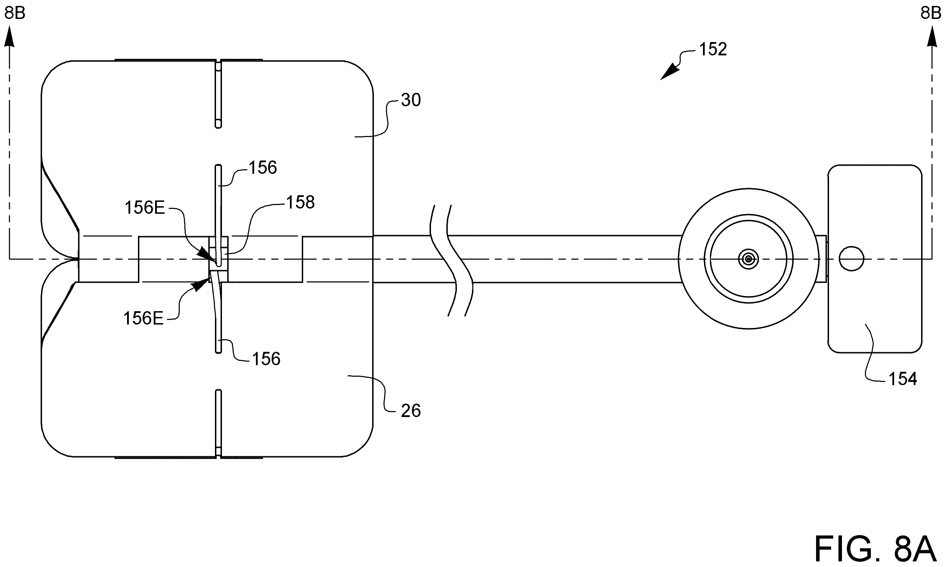

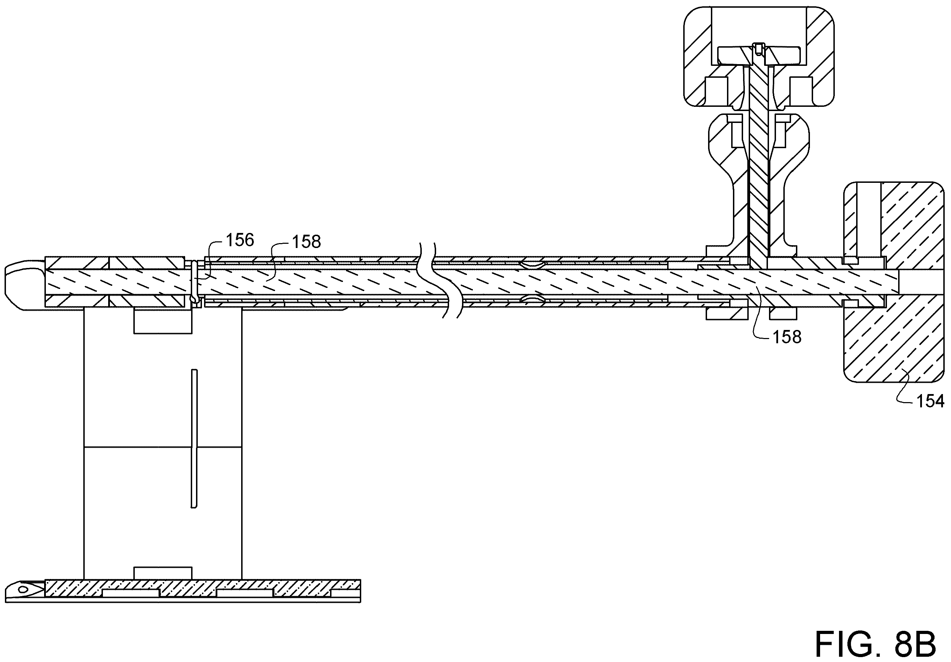

FIG. 7 is a perspective view of another embodiment of a cardiac retractor 152. This embodiment is similar to the previous embodiment, except that the tension knob 154 is coupled to the deployment cable 156 in a different manner. FIG. 8A is a top view of the cardiac retractor 152 of FIG. 7 showing cross-section line 8B-8B. In this view, it can be seen that the ends 156E of the deployment cable 156 are coupled directly to a tension rod 158 which passes within the fixed and keyed links 26, 30. The tension rod 158 is directly coupled to the tension knob 154 such that rotation of the tension knob 154 rotates the tension rod 158, thereby either drawing in or letting out the deployment cable 156. FIG. 8B is a side cross-sectional view of the cardiac retractor of FIG. 8A, taken along line 8B-8B.

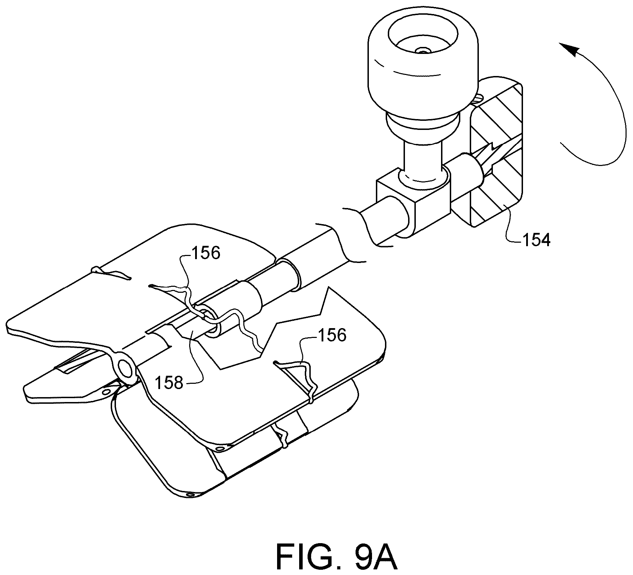

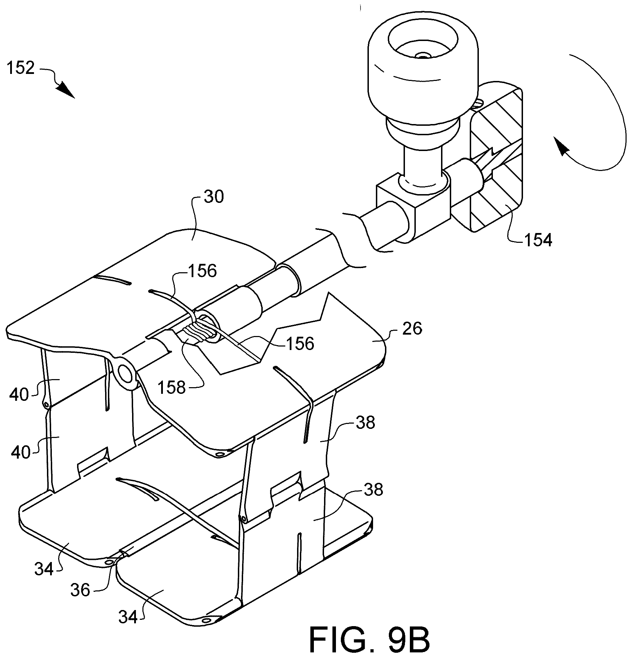

FIGS. 9A and 9B are both partial cross-sectional and partially exposed views which illustrate the deployment cable mechanism for the cardiac retractor embodiment of FIG. 7. In FIG. 9A, the tension knob 154 has been loosed, meaning the folded links are not caused to be deployed. In FIG. 9B, the tension knob has been tightened, causing the deployment cable 156 to wrap around the tension rod 158, thereby causing the deployment cable 156 to open the links 38, 38, 34, 36, 34, 40, and 40.

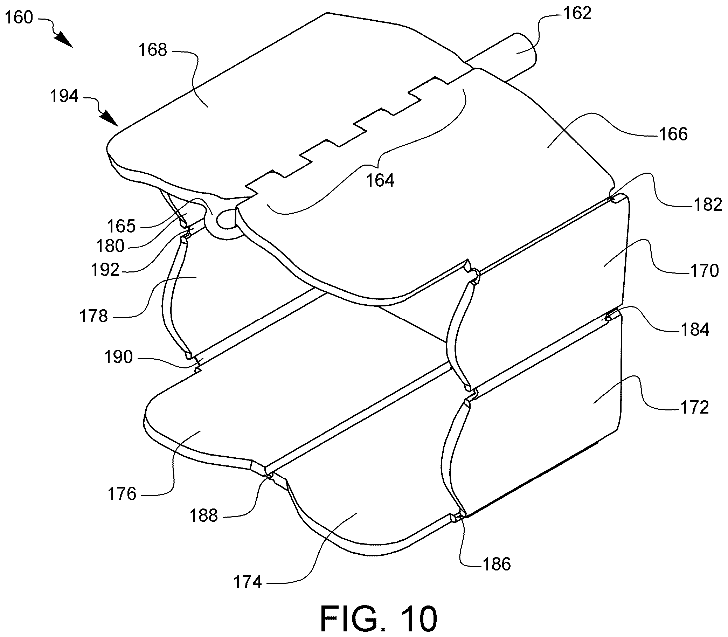

FIG. 10 is a perspective view of a distal end of another embodiment of a cardiac retractor. This embodiment of a distal end 160 of another embodiment of a cardiac retractor is fabricated using a single piece of material, most likely plastic. Other embodiments may use other materials capable of being formed in such a monolithic manner of a single piece having similar features as the embodiment described herein, for example, a variety of plastics, metals, elastomers, or combinations thereof. Alternate embodiments may include more than one single piece and be joined in a similar manner as embodiments described previously herein. The distal end 160 defines all features illustrated in FIG. 10, as it is of a singular piece. The distal end 160 defines a fixed collar 165 coupled to an outer tube 162, and a fixed top panel 168 also fixedly coupled to the outer tube 162. A keyed top panel 166 is also coupled to the outer tube 162, the keyed top panel 166 and the fixed top panel 168 along with the collar 165 forming a barrel hinge 164. The keyed top panel 166 is coupled to a side panel 170 by a living hinge 182, a second side panel 172 is coupled to side panel 170 by a living hinge 184, a bottom panel 174 is coupled to the side panel 172 by a living hinge 186, a second bottom panel 176 is coupled to the bottom panel 174 by a living hinge 188, a third side panel 178 is coupled to the second bottom panel 176 by a living hinge 190, a fourth side panel 180 is coupled to the third side panel 178 by a living hinge 192, and the fourth side panel 180 is coupled to the fixed top panel 168 by a living hinge 194, which is not visible here. The various living hinges 182, 184, 186, 188, 190, 192, 194 allow the various panels 166, 168, 170, 172, 174, 176, 178, 180 to fold into various stages of deployment as illustrated with previous embodiments of cardiac retractor described and depicted herein.

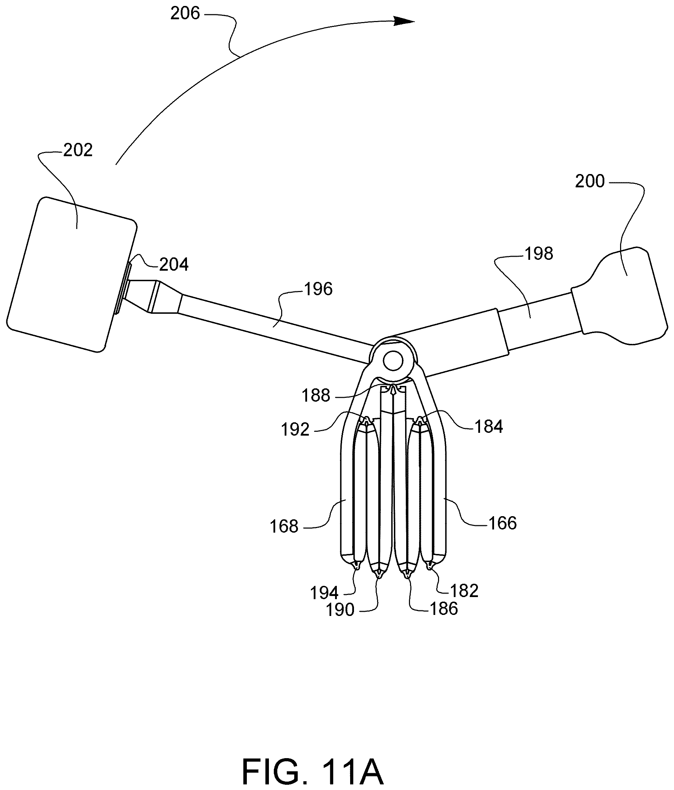

FIGS. 11A-11C are schematic elevational views of the distal end of the cardiac retractor of FIG. 10 illustrating various states of deployment of the cardiac retractor. These various stages of deployment are shown outside of an anatomical location, as compared to previously described embodiments for the purpose of clarity. FIG. 11A shows the cardiac retractor having its fixed key 200 rotated outward and having shaft 196 also in an outward position such that the fixed top panel 168 is rotated down towards the keyed top panel 166, and with the other links folded therebetween. The folded panels would fit within a cannula passage as previously shown, while the key hole is sized to hold the outer tube 162. Once the links have passed through the cannula, the outer tube 162 can be rotated within the cannula to orient the folded links as desired.

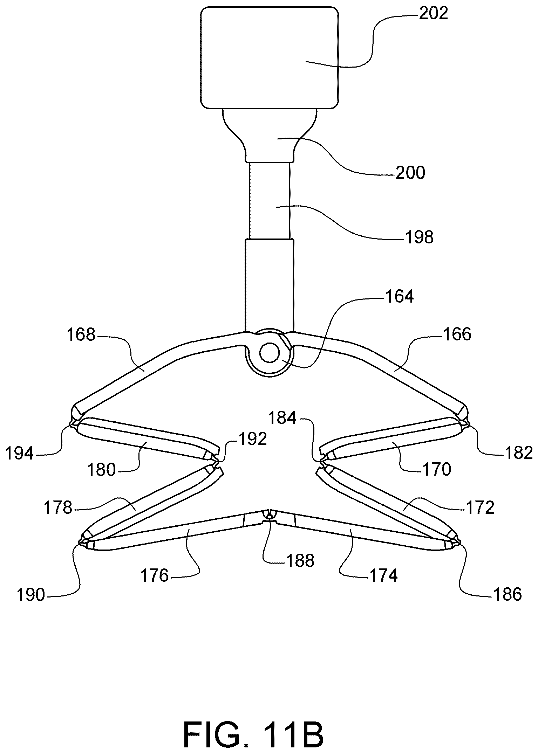

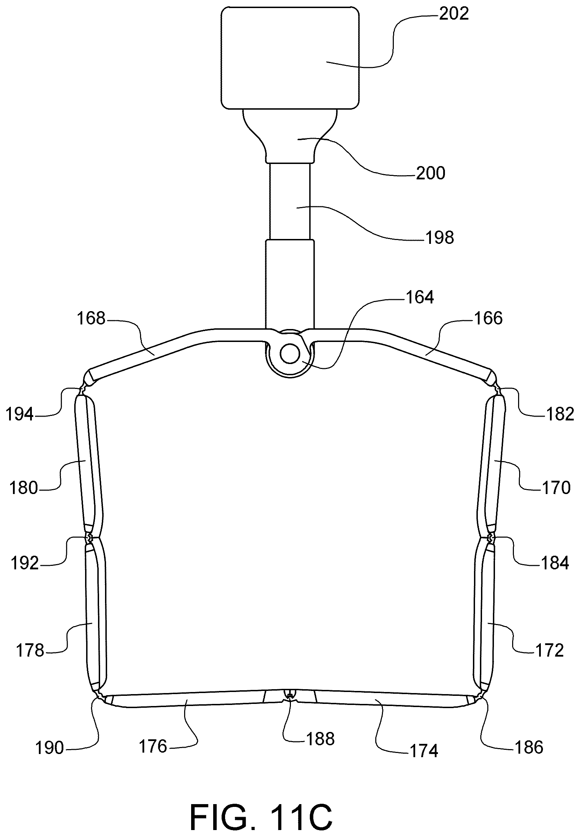

As shown in FIG. 11B, the fixed key 200 is then rotated in direction 206 into alignment with the fixed collar 198, and then, the coaxial nut 202 is threaded into onto threads 204 at the end of shaft 196 to hold the fixed key 200 in this partially deployed position. Once inside the patient (not shown here), this rotation of the fixed key 200 into alignment with the fixed collar 198 results in the keyed top panel 166 pivoting to the partially open/partially deployed position relative to the fixed top panel 168 as shown in FIG. 11B. A fully deployed position as illustrated in FIG. 11C can be achieved by manual manipulation by a surgeon, utilizing a grasper, a tenaculum or other surgical tools known to those skilled in the arts.

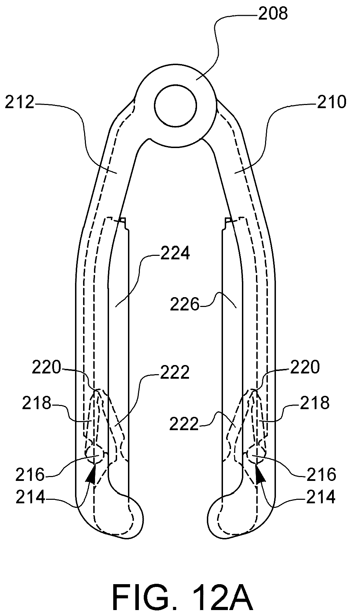

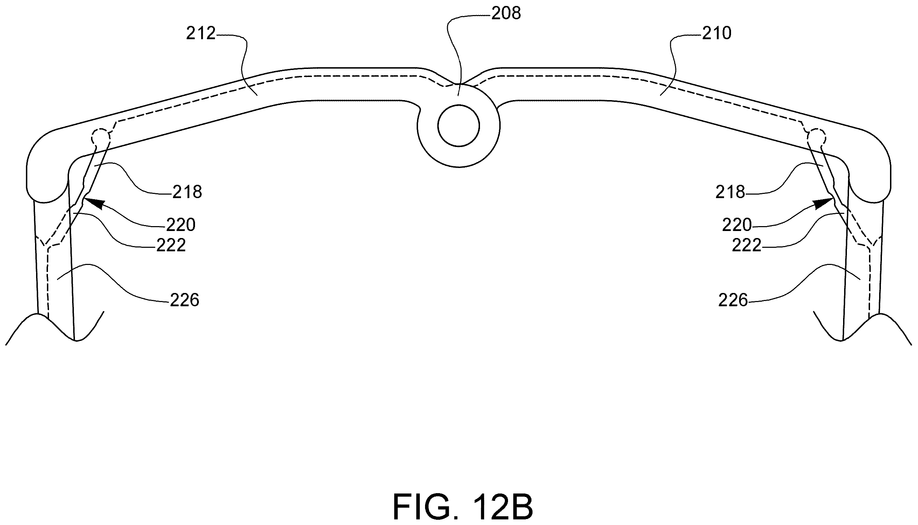

FIGS. 12A-12B are schematic elevational views of the distal end of another embodiment of a cardiac retractor, this embodiment having additional internal support arms. In the embodiment shown in FIGS. 12A and 12B, similar features described in regard to FIG. 10 are illustrated, but most of the side panels are removed from this view to focus on several support arms. The distal end of the cardiac retractor shown in FIG. 12A defines a barrel hinge 208, a top fixed panel 212, and a top keyed panel 210. A side panel 226 is coupled to the top keyed panel 210 and a side panel 224 is coupled to the top fixed panel 212. Coupled between the side panel 224 and top fixed panel 212 and coupled between the side panel 226 and top keyed panel 210, is a first support arm segment 218 coupled to a living support arm hinge 220 coupled to a second support arm segment 222. Each of the first support arm segments 218 are coupled directly to both top panels 210, 212 by a hinge pin 216 at the end of each first support arm segment 218 being snapped into a hinge knuckle 214 defined by each of the top panels 210, 212. FIG. 12B illustrates the distal end of the cardiac retractor in a fully deployed state. The support arms and their respective components, the first support arm segment 218, support arm hinge 220, and second support arm segment 222 are shown in a deployed and extended state. The function of the support arms is to provide support to between each top panel 210, 212 and each side panel 226, 224 until such time as a procedure requiring the use of a cardiac retractor is completed. The cardiac retractor may then be folded into a closed position and removed from the surgical site.

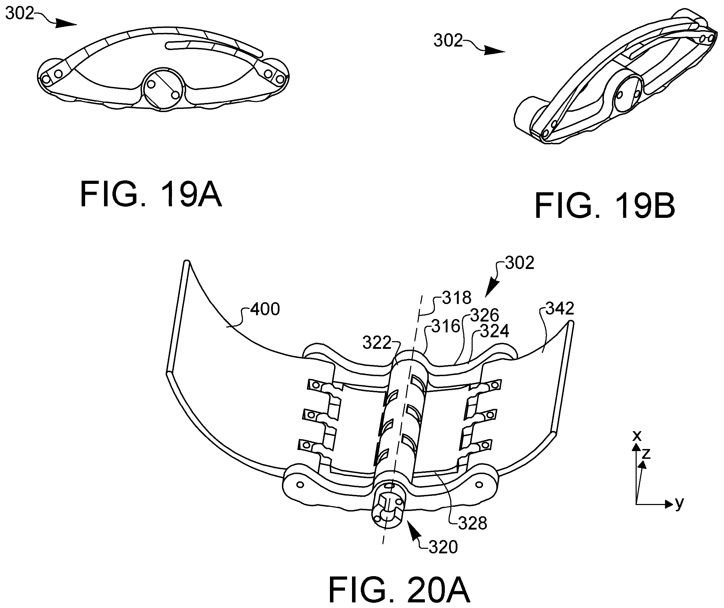

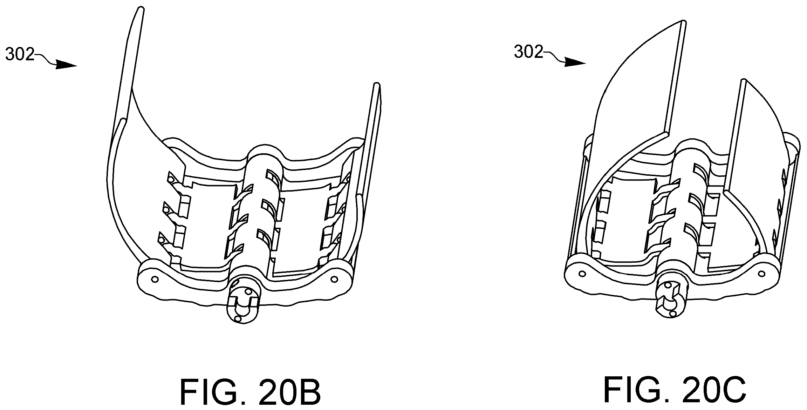

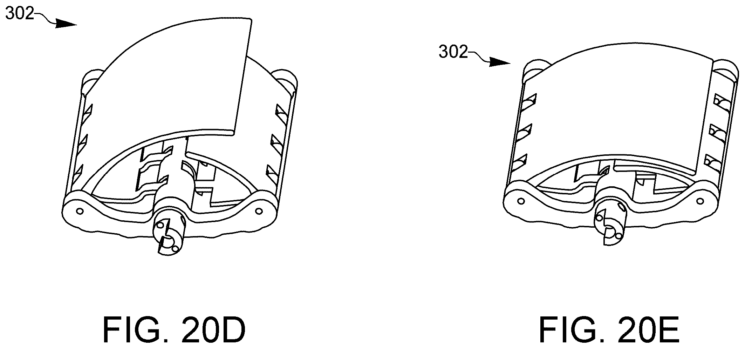

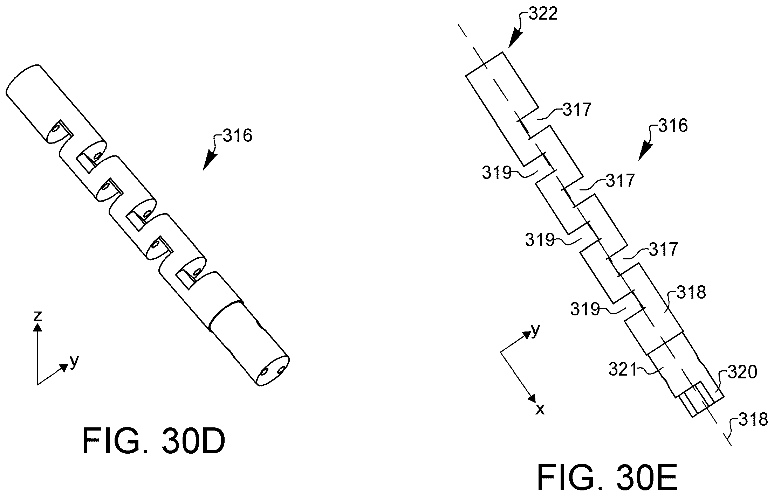

A further embodiment of a cardiac retractor device 300 that includes a cardiac retractor assembly 302 is illustrated in Figs. 13A to 13D. The cardiac retractor assembly 302 is illustrated in a retracted position in Figs. 13A to 13D and in an extended position in Fig. 20A. With reference to Figs. 30D and 30E, the cardiac retractor assembly 302 includes an elongated hub 316 that extends along a hub axis 318 from a proximal end 320 to a distal end 322, and the hub axis may extend parallel to the X-axis of the reference coordinate system of Figs. 30D and 30E. The hub 316 may be cylindrical or substantially cylindrical and may have an outer surface 321. The hub 316 may have one or more first recesses 317 formed in and along the outer surface 321 of the hub 316 in a first half of a plane that extends along the hub axis 318 and is parallel to the Z-X plane of the reference coordinate system of Figs. 30D and 30E. The hub 316 may also have one or more second recesses 319 formed in and along the outer surface 321 of the hub 316 in a second half of the plane that extends along the hub axis 318 and is parallel to the Z-X plane of the reference coordinate system of Figs. 30D and 30E, and first recesses 317 may alternate with the second recesses 319 along the hub axis 318. In some embodiments, the hub 316 may have three first recesses 317 and three second recesses 319.

With reference to Figs. 30B and 30C, a first hub aperture 360 may extend through the hub 316 parallel to and offset (in a first direction) from the hub axis 318 from the proximal end 320 to the distal end 322 of the hub 316. A portion of the first hub aperture 360 may extend through a corresponding two adjacent surfaces defining each of the one or more first recesses 317, and each of the two adjacent surfaces may be normal to the hub axis 318. A second hub aperture 362 may extend through the hub 316 parallel to and offset (in a second direction that is opposite to the first direction) from the hub axis 318 from the proximal end 320 to the distal end 322 of the hub 316. A portion of the second hub aperture 362 may extend through a corresponding two adjacent surfaces defining each of the one or more second recesses 319, and each of the two adjacent surfaces may be normal to the hub axis 318. The first hub aperture 360 may be 180° offset from the second hub aperture 362 when viewed along the hub axis 318. The hub 316 may be rotatably coupled to the base plate 304 such that the hub 316 rotates about the hub axis 318 relative to the base plate 304 from a first hub position (illustrated in Fig. 20A) to a second hub position (illustrated in Fig. 20E).

As illustrated in Figs. 29A to 29C, the cardiac retractor assembly 302 additionally includes the base plate 304 extending from a proximal end 306 to a distal end 308 along a base axis 310, and the base axis may be coaxially aligned with the hub axis 318. The base plate 304 may be further defined by a first lateral edge 312 and a second lateral edge 314 that are each offset from the base axis 310. Each of the first lateral edge 312 and the second lateral edge 314 may be linear or substantially linear and may be parallel to the base axis 310. A cylindrical guide 364 may extend along the base axis 310 from the proximal end 306 to the distal end 308, and may have an inner diameter that is slightly greater than an outer diameter of the hub 316 such that all of a portion of the hub 316 is rotatably disposed within the guide 364. So disposed, a distal portion of the hub 316 may extend from the distal end of the guide 364 and a proximal end of the hub 316 may extend from the proximal end of the guide 364. The guide 364 may have one or more first slots 366 that may overlap with the corresponding one of the one or more first recesses 317 of the hub 316, and the guide 364 may have one or more second slots 368 that may overlap with the corresponding one of the one or more second recesses 319 of the hub 316.

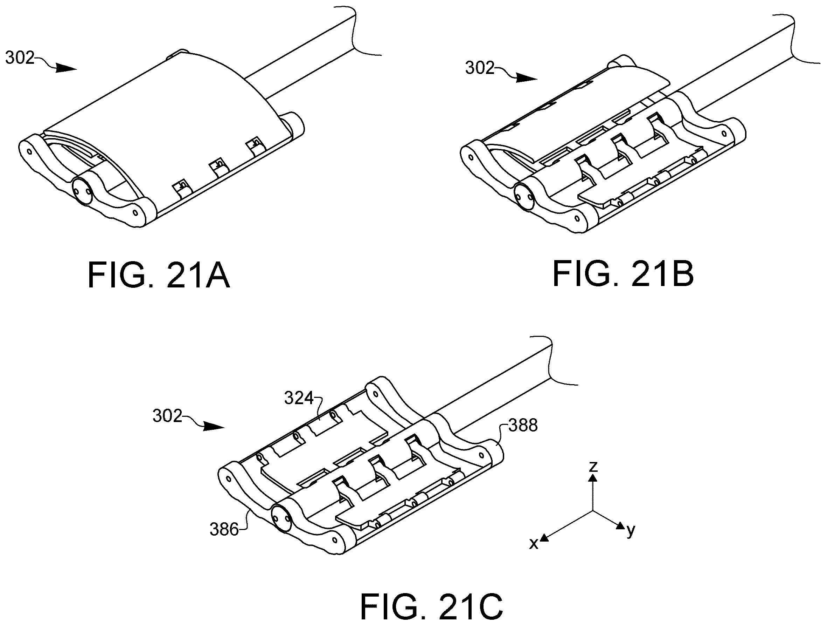

As illustrated in Fig. 21C, the cardiac retractor assembly 302 may additionally include a distal end plate 386 may be disposed along the distal end 308 of the base plate 304 and may extend from a first end to a second end along an axis that may extend parallel or substantially parallel to the Y-axis of the reference coordinate system of Fig. 29A (or to the hub axis 318), and the distal end plate 386 may be coupled to or fixed relative to the base plate 302. The cardiac retractor assembly 302 may also include a proximal end plate 388 that may be disposed along the proximal end 306 of the base plate 304 and may extend from a first end to a second end along an axis that may extend parallel or substantially parallel to the Y-axis of the reference coordinate system of Fig. 29A (or to the hub axis 318) and the proximal end plate 388 may be coupled to or fixed relative to the base plate 302.

Referring to Fig. 31, which is an exploded top view of the cardiac retractor assembly 302, the cardiac retractor assembly 302 additionally includes a first linkage member 324 that extends from a proximal end 326 to a distal end 328 along a first linkage member axis 330 that may be parallel to the X-axis of the reference coordinate system of Fig. 21C, the first linkage member 324 having a first lateral edge 332 and a second lateral edge 334 that is each offset from the first linkage member axis 330. Each of the first lateral edge 332 and the second lateral edge 334 may be linear or substantially linear and may be parallel to the first linkage member axis 330. The first linkage member 324 may be planar or substantially planar. In some embodiments, the first linkage member 324 may have a cross-sectional shape in the Y-Z plane of the reference coordinate system of Fig. 21C that may be cambered from the first lateral edge 332 to the second lateral edge 334, and the cross-sectional shape may be uniform or substantially uniform along the first linkage member axis 330.

A first portion of the first linkage member 324 may be pivotably coupled to a first portion of the hub 316 such that the first portion of the first linkage member 324 pivots about an axis 340 that is offset from and parallel to the hub axis 318. In particular, one or more first projections 370 may extend from the first lateral edge 332 in a direction that is parallel or generally parallel to the Y-axis of the reference coordinate system of Fig. 31, and each of the one or more first projections 370 may have an aperture 372 that extends parallel to the X-axis of the reference coordinate system of Fig. 31. Each of the one or more first projections 370 may be received into a corresponding one of the first slots 366 of the guide 364 and one of the first recesses 317 of the hub 316. The aperture 372 of each of the one or more first projections 370 may receive a portion of a first axle 374 that extends parallel to the X-axis, and a portion of the first axle 374 may be received in the first hub aperture 360 such that the first linkage member 324 is pivotable relative to the base plate 304 and the hub 316 about the first axle 374. In addition, one or more second projections 374 may extend from the second lateral edge 334 in a direction that is parallel or generally parallel to the Y-axis of the reference coordinate system of Fig. 31, and that is opposite to the one or more first projections 370. Each of the one or more second projections 374 may have an aperture 378 that extends parallel to the X-axis of the reference coordinate system of Fig. 21C.

The cardiac retractor assembly 302 also includes a first blade 342 that extends from a proximal end 344 to a distal end 346 along a first blade axis 348 that may be parallel to the X-axis of the reference coordinate system of Fig. 20A, the first blade 342 having a first lateral edge 350 and a second lateral edge 352 that is each offset from the first blade axis 348. Each of the first lateral edge 350 and the second lateral edge 352 may be linear or substantially linear and may be parallel to the first blade axis 348. The first blade 342 may have a cross-sectional shape in the Y-Z plane of the reference coordinate system of Fig. 20A that may be cambered from the first lateral edge 350 to the second lateral edge 352, and the cross-sectional shape may be uniform or substantially uniform along the first blade axis 348.

A first portion of the first blade 342 may be pivotably coupled to a second portion of the first linkage member 324 that is disposed at or adjacent to the second lateral edge 334 of the first linkage member 324 such that the first portion of the first blade 342 pivots about a second axis that is parallel to the hub axis 318. In particular, one or more first blade slots 378 may extend inwards from the first lateral edge 350, and a first aperture 380 may extend through a corresponding pair of opposing surfaces that partially define each of the first blade slots 378, and the corresponding pair of opposing surfaces may be planar or substantially planar and may extend normal to the X-axis of the reference coordinate system of Fig. 31. Each of the first apertures 380 may extend parallel to the X-axis of the reference coordinate system of Fig. 31. Each of the one or more second projections 374 may be received into a corresponding one of the first blade slots 378, and an axis of the first apertures 380 and an aperture of the aperture 378 of the second projections 374 may be coaxially aligned and receive a second axle 382 such that the first blade 342 rotates about the first linkage member 324 about the second axle 382.

A blade pivot aperture 384 may extend through the first blade 342 along a first blade pivot axis 373 that may be along or offset from the first blade axis 348. In some embodiments, the blade pivot aperture 384 may extend through the corresponding pair of opposing surfaces that partially define each of the first blade slots 378, and the first aperture 380 may be disposed between the blade pivot aperture 384 and the first lateral edge 350 of the first blade 342. A third axle 390 may extend from a proximal end to a distal end along a third axle axis that is aligned with the first blade pivot axis 373 such that all or a portion of the third axle 390 is disposed within the blade pivot aperture 384. The distal end of the third axle 390 may be fixedly secured to a portion of the distal end plate 386 and the proximal end of the third axle 390 may be fixedly secured to a portion of the proximal end plate 388 such that the first blade 342 pivots about the third axle 390 relative to the base plate 304.

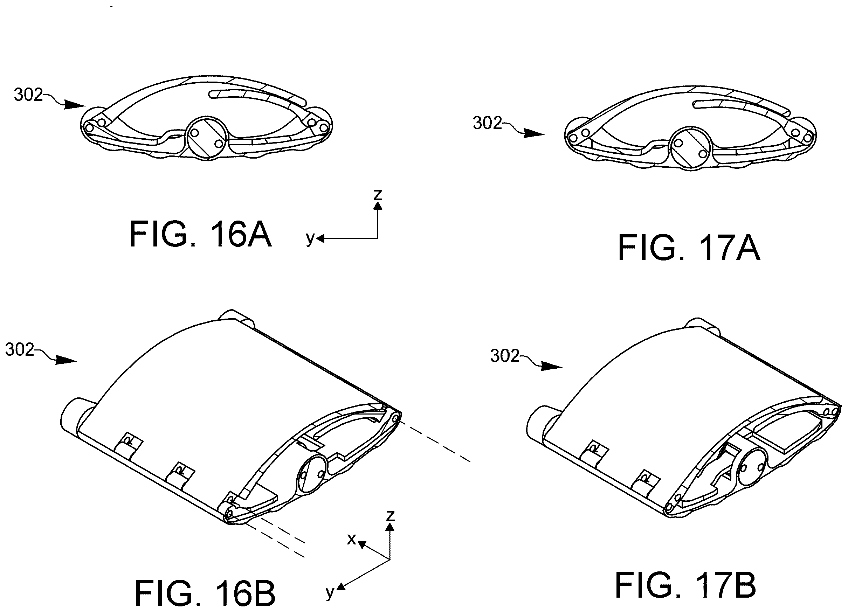

Thus, as illustrated in Figs. 20A to 20E, when the hub 316 is in the second hub position (illustrated in Fig. 20E and 16A), the first linkage member 324 is in an extended position, and the first blade 342 is in the retracted position, illustrated in Figs. 16A and 20E. In this retracted position, the second lateral edge 352 of the first blade 342 may be disposed adjacent to a portion of the guide 364.

When the hub 316 is rotated about the hub axis 318 in a first direction (e.g., a clockwise direction relative to the cross-sectional view of Fig. 16A) from the second hub position towards the first hub position, the first linkage member 324 is displaced towards the second lateral edge 314 of the base member 304 substantially along the Y-axis of the reference coordinate system of Figs. 16A and 31 and thus translates from an extended position towards a retracted position. Because the second axle 382 displaces towards the hub axis 318 with the first linkage member 324, the inner lateral portion of the first blade 342 that receives corresponding portion of the second axle 382 also displaces with the first linkage member 324, which rotates the first blade 342 about the third axle 390 such that the first blade 342 begins to rotate in the clockwise direction relative to the cross-sectional view of Fig. 16A. When the hub is rotated into the first hub position (illustrated in Fig. 20A), the first linkage member 324 is displaced to the retracted position, thereby rotating the first blade 342 about the third axle 390 into the extended position of Fig. 20A.

Referring to Fig. 31, which is an exploded top view of the cardiac retractor assembly 302, the cardiac retractor assembly 302 additionally includes a second linkage member 392 that extends from a proximal end 394 to a distal end 396 along a second linkage member axis 398 that may be parallel to the X-axis of the reference coordinate system of Figs. 21C and 30, the second linkage member 392 having a first lateral edge 404 and a second lateral edge 406 that is each offset from the second linkage member axis 398. Each of the first lateral edge 404 and the second lateral edge 406 may be linear or substantially linear and may be parallel to the second linkage member axis 398. The second linkage member 392 may be planar or substantially planar. In some embodiments, the second linkage member 392 may have a cross-sectional shape in the Y-Z plane of the reference coordinate system of Fig. 21C that may be cambered from the first lateral edge 404 to the second lateral edge 406, and the cross-sectional shape may be uniform or substantially uniform along the second linkage member axis 398.

A first portion of the second linkage member 392 may be pivotably coupled to a second portion of the hub 316 such that the first portion of the second linkage member 392 pivots about an axis that is offset from and parallel to the hub axis 318. In particular, one or more first projections 408 may extend from the first lateral edge 404 in a direction that is parallel or generally parallel to the Y-axis of the reference coordinate system of Fig. 31, and each of the one or more first projections 408 may be identical or substantially identical to the one or more first projections 370 of the first linkage member 324. Each of the one or more first projections 408 may have an aperture 410 that extends parallel to the X-axis of the reference coordinate system of Fig. 31. Each of the one or more first projections 408 may be received into a corresponding one of the second slots 368 of the guide 364 and one of the second recesses 319 of the hub 316. The aperture 410 of each of the one or more first projections 408 may receive a portion of a fourth axle 412 that extends parallel to the X-axis, and a portion of the fourth axle 412 may be received in the second hub aperture 362 such that the second linkage member 392 is pivotable relative to the base plate 304 and the hub 316 about the fourth axle 412. In addition, one or more second projections 414 may extend from the second lateral edge 406 in a direction that is parallel or generally parallel to the Y-axis of the reference coordinate system of Fig. 31, and that is opposite to the one or more first projections 408. Each of the one or more second projections 414 may have an aperture 416 that extends parallel to the X-axis of the reference coordinate system of Fig. 31.

The cardiac retractor assembly 302 also includes a second blade 400 that extends from a proximal end 418 to a distal end 420 along a second blade axis 402 that may be parallel to the X-axis of the reference coordinate system of Fig. 31, the second blade 400 having a first lateral edge 422 and a second lateral edge 424 that is each offset from the second blade axis 402. Each of the first lateral edge 350 and the second lateral edge 352 may be linear or substantially linear and may be parallel to the second blade axis 402. The second blade 400 may have a cross-sectional shape in the Y-Z plane of the reference coordinate system of Fig. 20A that may be cambered from the first lateral edge 422 to the second lateral edge 424, and the cross-sectional shape may be uniform or substantially uniform along the second blade axis 402.

A first portion of the second blade 400 may be pivotably coupled to a second portion of the second linkage member 392 that is disposed at or adjacent to the second lateral edge 334 of the second linkage member 392 such that the first portion of the second blade 400 pivots about a second axis that is parallel to the hub axis 318. In particular, one or more second blade slots 426 may extend inwards from the first lateral edge 422, and a second aperture 428 may extend through a corresponding pair of opposing surfaces that partially define each of the second blade slots 426, and the corresponding pair of opposing surfaces may be planar or substantially planar and may extend normal to the X-axis of the reference coordinate system of Fig. 31. Each of the second apertures 428 may extend parallel to the X-axis of the reference coordinate system of Fig. 31. Each of the one or more second projections 414 of the second linkage member 392 may be received into a corresponding one of the second blade slots 426, and an axis of the second apertures 428 and an axis of the aperture 416 of the second projections 374 may be coaxially aligned and receive a fifth axle 430 such that the second blade 400 rotates relative to the second linkage member 392 about the fifth axle 430.

A blade pivot aperture 432 may extend through the second blade 400 along a second blade pivot axis 434 that may be along or offset from the second blade axis 402. In some embodiments, the blade pivot aperture 432 may extend through the corresponding pair of opposing surfaces that partially define each of the second blade slots 426, and the second aperture 428 may be disposed between the blade pivot aperture 432 and the first lateral edge 422 of the second blade 400. A sixth axle 436 may extend from a proximal end to a distal end along a sixth axle axis that is aligned with the second blade pivot axis 434 such that all or a portion of the sixth axle 436 is disposed within the blade pivot aperture 432. The distal end of the sixth axle 436 may be fixedly secured to a portion of the distal end plate 386 and the proximal end of the sixth axle 436 may be fixedly secured to a portion of the proximal end plate 388 such that the second blade 400 pivots about the sixth axle 436 relative to the base plate 304.

Thus, as illustrated in Figs. 20A to 20E, when the hub 316 is in the second hub position (illustrated in Fig. 20E and 16A), the second linkage member 392 is in an extended position, and the second blade 400 (and the retractor end assembly 302) is in the retracted position, illustrated in Figs. 16A and 20E. In this retracted position, the second lateral edge 424 of the second blade 400 may be disposed adjacent to or aligned with the first lateral edge 312 of the base plate 304.

When the hub 316 is rotated about the hub axis 318 in the first direction (e.g., a clockwise direction relative to the cross-sectional view of Fig. 16A) from the second hub position towards the first hub position, the second linkage member 392 is displaced towards the first lateral edge 312 of the base member 304 substantially along the Y-axis of the reference coordinate system of Fig. 16A and 31 and thus translates from an extended position towards a retracted position. Because the fifth axle 430 displaces towards the hub axis 318 with the second linkage member 392, the inner lateral portion of the second blade 400 that receives corresponding portion of the fifth axle 430 also displaces with the second linkage member 392, which rotates the second blade 400 about the sixth axle 436 such that the second blade 400 begins to rotate in the counter-clockwise direction relative to the cross-sectional view of Fig. 16A. When the hub 316 is rotated into the first hub position (illustrated in Fig. 20A), the second linkage member 392 is displaced into the retracted position, thereby rotating the second blade 400 about the sixth axle 390 into the extended position of Fig. 20A. The angular displacement of the hub 316 about the hub axis 318 between the first hub position and the second hub position may be any suitable angular value, such as an angular distance of 40° to 210°, or such as 150° to 200°, or such as 170° to 190°, or such as 70° to 110°, or such as 80° to 100°.

To actuate or displace the retractor end assembly 302 from the extended position to the retracted position, the process is reversed, and the hub 316 is rotated from the first hub position (illustrated in Fig. 20A) to the second hub position (illustrated in Figs. 16A and 20E).

As illustrated in Figs. 13A to 13D, the cardiac retractor device 300 may include a shaft portion 438 that may extend from a proximal end 440 to a distal end 442 along a shaft axis 444, and the distal end 442 of the shaft portion 438 may be coupled to a proximal portion of the cardiac retractor assembly 302 . The shaft portion 438 may be extendable and retractable along the shaft axis 444 to change the length of the shaft portion 438. In particular, the shaft portion 438 may include a first shaft portion 446 that may extend from a proximal end 448 to a distal end 450 along the shaft axis 444, and the distal end 450 may correspond to the distal end 442 of the shaft portion 438. The first shaft portion 446 may include a hollow tube portion having a first diameter. The shaft portion 438 may further include a second shaft portion 452 that may extend from a proximal end 454 to a distal end 456 along the shaft axis 444, and the proximal end 452 may correspond to the proximal end 440 of the shaft portion 438. The second shaft portion 452 may include a hollow tube portion having a second diameter that is less than the first diameter such that the distal end 456 of the second shaft portion 452 can be inserted into the open proximal end 448 of the first shaft portion 438 and advanced distally towards the distal end 450 of the first shaft portion 446. Fig. 13A illustrated the shaft portion 438 in a fully extended position, but the shaft portion 438 may be in a fully retracted position (in which the distal end 456 of the second shaft portion 452 is adjacent to the distal end 450 of the first shaft portion 446). The shaft portion 438 may be in any of a plurality of intermediate positions in which the distal end 456 of the second shaft portion 452 is disposed at various positions between the proximal end 448 and the distal end 450 of the first shaft portion 446.

A locking collar 458 may be disposed at the proximal end 448 of the first shaft portion 438, and the locking collar 458 may have a lever 450 having an end portion that is biased into engagement with one of a plurality of slots 459 formed on an outer surface of the second shaft portion 452 to lock the second shaft portion 452 in a desired position relative to the first shaft portion 446.

The cardiac retractor device 300 may also include an actuation assembly 461 that is configured to actuate or displace the retractor end assembly 302 between the retracted position and the expanded position. The actuation assembly 461 may include a first transmission rod 462 that may be disposed within all or a portion of an interior portion of the first shaft portion 446, and the first transmission rod 462 may extend from a proximal end 464 to a distal end 466 along the shaft axis 444. As illustrated in Fig. 36, in which a portion of a collar is omitted for clarity, the distal end 466 of the first transmission rod 462 may be fixedly coupled to the proximal end 320 of the hub 316 such that the first transmission rod 462 does not displace along the shaft axis 444 relative to the retractor end assembly 302, but such that a rotation of the first transmission rod 462 about the shaft axis 444 results in a corresponding rotation of the hub 316 about the hub axis 318, which may be coaxially aligned with the shaft axis 444.

The first transmission rod 462 may have a non-circular cross-sectional shape when viewed normal to the shaft axis 444 (as illustrated in Figs 25 and 34B), and the cross-section may have a generally crescent shape that includes one or more protrusions and one or more recesses. For example, the cross-sectional shape may include an outer edge that is a segment of a circle, and a non-linear interior edge that extends from a first end of the outer edge to a second end of the outer edge. The interior edge may define a central recess edge that may be a segment of a circle, and a pair of protrusions that surround the central recess edge. The cross-sectional shape may be uniform from the proximal end 464 to the distal end 466, thereby defining a central recess that extends along an exterior surface of the first transmission rod 462 from the proximal end 464 to the distal end 466.

The actuation assembly 461 may also include a second transmission rod 468 that may be disposed within all or a portion of an interior portion of the second shaft portion 452, and the second transmission rod 468 may extend from a proximal end 470 to a distal end 472 along the shaft axis 444. As illustrated in the cross-sectional view of Fig. 23, the proximal end 470 of the second transmission rod 468 may be fixedly coupled to a gear portion 474 of the transmission assembly 460 (that will be described in more detail below) such that when the gear portion 474 is rotated about the shaft axis 444, the second transmission rod 468 also rotates about the shaft axis 444.

The second transmission rod 468 may have a non-circular cross-sectional shape when viewed normal to the shaft axis 444 (as illustrated in Figs 27 and 34B), and the cross-section may have a generally crescent shape that includes one or more protrusions and one or more recesses. For example, with reference to Fig. 34B, the cross-sectional shape may include an outer edge that is a segment of a circle, and a non-linear interior edge that extends from a first end of the outer edge to a second end of the outer edge. The interior edge may define a central protrusion edge that may be a segment of a circle, and a pair of recesses that surround the central protrusion edge. The cross-sectional shape may be uniform from the proximal end 470 to the distal end 466, thereby defining a central protrusion that extends along an exterior surface of the second transmission rod 468 from the proximal end 470 to the distal end 472. The non-linear interior edge may be complementary or a mirror of the non-linear interior edge of the first transmission rod 462. Accordingly, the central protrusion of the second transmission rod 468 may be received into the central recess of the first transmission rod 462, and the pair of protrusions of the first transmission rod 462 may be received into the pair of recesses of the second transmission rod 468. So configured, the second transmission rod 468 may translate along the shaft axis 444 relative to the first transmission rod 462 when the second shaft portion 452 is displaced relative to the first shaft portion 446. However, this configuration also provides for rotation of the first transmission rod 462 (and the hub 316) about the shaft axis 444 when the gear portion 474 rotates the second transmission rod 468 about the shaft axis 444. The second shaft portion 452 and the second transmission rod 468 may be dimensioned such that the distal end 472 of the second transmission rod 468 is distal to the proximal end 464 of the first transmission rod 462 when the shaft portion is in the fully extended configuration of Fig. 13A, but still allows for the transmission of rotation from the second transmission rod 468 to the first transmission rod 462 with sufficient torque to rotate the hub 316 between the first hub position and the second hub position. The second shaft portion 452 and the second transmission rod 468 may also be dimensioned such that the distal end 472 of the second transmission rod 468 is at or proximal to the distal end 320 of the hub 316 when the shaft portion is in a fully retracted configuration (illustrated in Fig. 32A and 32C).

As previously explained, the actuation assembly 461 may also include a transmission assembly 460 coupled to a portion of the shaft portion 438, such as a portion of the shaft portion 438 at or adjacent to the proximal end 440 of the shaft portion 438 or at or adjacent to the proximal end 454 of the second shaft portion 452. As illustrated in the views of Figs. 22A and 22B (where a rear portion of the housing is emitted for clarity) and the cross-sectional view of Fig. 23,1 the transmission assembly 460 includes the gear portion 474 fixedly coupled to the proximal end 470 of the second transmission rod 468. The gear portion 474 may include a plurality of teeth that may extend around all or a portion of the circumference of the gear portion 474, and the plurality of teeth may be engaged by a worm drive 476 that may be mounted to a housing 478 that encloses the gear portion 474, the proximal end 470 of the second transmission rod 468, and the worm drive 476. A stem may be coupled to the worm drive 476, and a portion of the stem may extend through an aperture in the housing, and a knob 479 may be disposed on an end portion of the stem. Thus, a rotation of the knob in a first direction about an axis of the stem may result in a corresponding rotation of the second transmission rod 468 in a first direction about the shaft axis 444 (which is 90° offset from the stem axis). Similarly, a rotation of the knob in a second direction about the axis of the stem may result in a corresponding rotation of the second transmission rod 468 in a second direction about the shaft axis 444. The length of the worm drive 476 and/or the circumferential length of the plurality of teeth of the gear portion 474 may control the amount of rotation of the second transmission rod 468 about the shaft axis 444.Furhter, because neither the first transmission rod 462 nor the second transmission rod 468 are fixedly coupled to the first shaft portion 446 or the second shaft portion 452, the first transmission rod 462 and the second transmission rod 468 can rotated relative to the first shaft portion 446 and the second shaft portion 452 in any configuration of the shaft portion 438.

A further embodiment of a cardiac retractor device 500 illustrated in Figs. 32A to 32C. The embodiment of the cardiac retractor device 500 may include the cardiac retractor assembly 302 and shaft portion 438 illustrated in Figs. 13A to 13D but may have a different embodiment of an actuation assembly 502. The actuation assembly 502 may include the first transmission rod 462 (see FIG. 25) and the second transmission rod 468 (see FIGS. 26 to 28 and 32E) previously disclosed. Instead of a gear portion 474, a portion of a collar portion 504 may be fixedly coupled to the proximal end 470 of the second transmission rod 468 (or the first transmission rod 462) such that a rotation of the collar portion 504 about the shaft axis 444 results in a corresponding rotation of the second transmission rod 468 (or the first transmission rod 462) about the shaft axis 444, thereby displacing the cardiac retractor assembly 302 between the retracted position (of FIG. 32C) and the extended position (of Fig. 20A), as will be described in more detail below.

The actuation assembly 502 may also include an elongated housing 506 that may extend from a first end 508 to a second end 510 along a housing axis 512 that may be normal to the shaft axis 444, and the housing axis may extend along the Z-axis of the reference coordinate system of Figs. 32A and 32B. The collar portion 504 may be rotatably coupled to a portion of the housing 506 at or adjacent to the second end 510. The housing 506 may include a central bore 511 that may extend from the first end 508 to the second end 510.

With reference to the cross-sectional view of FIG 32D, the actuation assembly 502 may further include an elongated linear actuator 514 that may extend from a first end 516 to a second end 518 along the housing axis 512. The linear actuator 514 may be a single, unitary component or may be an assembly of two or more components that are coupled to form the linear actuator 514. A portion of the linear actuator 514 may be disposed in the central bore 511 of the housing 506, and a threaded portion 530 of an exterior surface of the linear actuator 514 may be threaded and may engage a threaded portion 531 of an internal surface of the housing 506 such that when the linear actuator 514 is rotated about the housing axis 512, the linear actuator 514 translates along the housing axis 512. The first end 516 of the linear actuator 514 may be disposed external to the central bore 511 of the housing 506, and a knob portion 532 may extend from the first end 516 towards the second end 518, with the entire knob portion 532 being external to the central bore 511 of the housing 506 such that the knob portion 532 may be rotated by the hand of a suer during a procedure to linearly displace the linear actuator 514.

The actuation assembly 502 may further include a link 520 that extends from a first end 522 to a second end 524. The first end 522 may be pivotably coupled to a portion of the linear actuator 514 at or adjacent to the second end 518, and the second end 524 may be pivotably coupled to a portion of the collar portion 504 (e.g., a recess formed in a portion of the collar portion 504) that is offset from the shaft axis 444. Thus, as the linear actuator 514 displaces towards the shaft axis 444 (or the second end 510 of the housing 506), such as when a user rotates the knob portion 532 in a first rotational direction about the housing axis 512, the second end 524 of the link 520 rotates the collar portion 504 and the second transmission rod 468 (or the first transmission rod 462) in a first direction. Thus, in one embodiment, the collar 504 may begin the rotation with the link 520 in the position illustrated in FIG. 32G, in which the cardiac retractor assembly 302 is in the retracted position of FIG. 32C, rotate through the intermediate position of FIG. 32H, in which the cardiac retractor assembly 302 is in the intermediate position of FIG. 20C,and end in the position of FIG. 32I, in which the cardiac retractor assembly 302 is in the extended position of Fig. 20A, as previously described.

Additionally, as the linear actuator 514 displaces away from the shaft axis 444 (or the second end 510 of the housing 506), such as when a user rotates the knob portion 532 in a second rotational direction about the housing axis 512, the second end 524 of the link 520 rotates the collar portion 504 and the second transmission rod 468 (or the first transmission rod 462) in a second direction. Thus, in one embodiment, the collar 504 may begin the rotation with the link 520 in the position illustrated in FIG. 32I, in which the cardiac retractor assembly 302 is in the extended position of FIG. 20A, rotate through the intermediate position of FIG. 32H, in which the cardiac retractor assembly 302 is in the intermediate position of FIG. 20C,and end in the FIG. 32G, in which the cardiac retractor assembly 302 is in the retracted position of Fig. 32C, as previously described.

In other embodiments, when a user rotates the knob portion 532 in the first rotational direction about the housing axis 512, the collar 504 may begin the rotation with the link 520 in the position illustrated in FIG. 32I, in which the cardiac retractor assembly 302 is in the retracted position of FIG. 32C, rotate through the intermediate position of FIG. 32H, in which the cardiac retractor assembly 302 is in the intermediate position of FIG. 20C,and end in the position of FIG. 32G, in which the cardiac retractor assembly 302 is in the extended position of Fig. 20A, as previously described. In this other embodiment, when a user rotates the knob portion 532 in the second rotational direction about the housing axis 512, the collar 504 may begin the rotation with the link 520 in the position illustrated in FIG. 32G, in which the cardiac retractor assembly 302 is in the extended position of FIG. 20A, rotate through the intermediate position of FIG. 32H, in which the cardiac retractor assembly 302 is in the intermediate position of FIG. 20C,and end in the FIG. 32I, in which the cardiac retractor assembly 302 is in the retracted position of Fig. 32C, as previously described.

As illustrated in Figs. 32G, the link 520 may have a slot (not shown) that defines a cam path that receives a pin fixed to the housing 506 such that the second end 524 of the link 520 is not aligned (e.g., along the housing axis 512) with the shaft axis 444 as it rotates the collar portion 504.

Various advantages of a cardiac retractor have been discussed above. Embodiments discussed herein have been described by way of example in this specification. It will be apparent to those skilled in the art that the forgoing detailed disclosure is intended to be presented by way of example only, and is not limiting. Various alterations, improvements, and modifications will occur and are intended to those skilled in the art, though not expressly stated herein. These alterations, improvements, and modifications are intended to be suggested hereby, and are within the spirit and the scope of the claimed invention. Additionally, the recited order of processing elements or sequences, or the use of numbers, letters, or other designations therefore, is not intended to limit the claims to any order, except as may be specified in the claims. Accordingly, the invention is limited only by the following claims and equivalents thereto.

## Claims

1. A cardiac retractor assembly (302), comprising:
a base plate (302, 304) extending from a proximal end (306, 320, 326, 344, 394, 418, 440, 448, 452, 454, 464, 470) to a distal end (160, 308, 320, 322, 328, 346, 396, 420, 442, 450, 456, 466, 472) along a base axis (310), the base plate (302, 304) having a first lateral edge (312, 332, 350, 404, 422) and a second lateral edge (314, 334, 352, 406, 424) that are each offset from the base axis (310);
an elongated hub (316) that extends along a hub axis (318) from a proximal end (306, 320, 326, 344, 394, 418, 440, 448, 452, 454, 464, 470) to a distal end (160, 308, 320, 322, 328, 346, 396, 420, 442, 450, 456, 466, 472), the hub (316) being rotatably coupled to the base plate (302, 304) such that the hub (316) rotates about the hub axis (318) relative to the base plate (302, 304) from a first hub (316) position to a second hub (316) position;
a first linkage member (324) extending from a proximal end (306, 320, 326, 344, 394, 418, 440, 448, 452, 454, 464, 470) to a distal end (160, 308, 320, 322, 328, 346, 396, 420, 442, 450, 456, 466, 472) along a first linkage member axis (330), the first linkage member (324) having a first lateral end and a second lateral end that is each offset from the first linkage member axis (330), wherein a first portion of the first linkage member (324) is pivotably coupled to a first portion of the hub (316) such that the first portion of the first linkage member (324) pivots about an axis (32, 340) that is offset from and parallel to the hub axis (318);
a first blade (342) extending from a proximal end (306, 320, 326, 344, 394, 418, 440, 448, 452, 454, 464, 470) to a distal end (160, 308, 320, 322, 328, 346, 396, 420, 442, 450, 456, 466, 472) along a first blade axis (348), the first blade (342) having a first lateral end and a second lateral end that is each offset from the first blade axis (348), wherein:
(a) a first portion of the first blade (342) is pivotably coupled to second portion of the first linkage member (324) that is disposed at or adjacent to the second lateral end of the first linkage member (324) such that the first portion of the first blade (342) pivots about an axis (32, 340) that is parallel to the hub axis (318), and
(b) a second portion of the first blade (342) is pivotable about a first blade pivot axis (373) that is fixed relative to the base plate (302, 304), the first blade pivot axis (373) being disposed between the first lateral end of the first blade (342) and the second lateral end of the first blade (342) such that (1) when the hub (316) rotates about the hub axis (318) from the first hub (316) position to the second hub (316) position, the first blade (342) rotates about the first blade pivot axis (373) from a first blade (342) closed position to a first blade (342) open position, and (2) when the hub (316) rotates about the hub axis (318) from the second hub (316) position to the first hub (316) position, the first blade (342) rotates about the first blade pivot axis (373) from the first blade (342) open position to the first blade (342) closed position;
a second linkage member (392) extending from a proximal end (306, 320, 326, 344, 394, 418, 440, 448, 452, 454, 464, 470) to a distal end (160, 308, 320, 322, 328, 346, 396, 420, 442, 450, 456, 466, 472) along a second linkage member axis (398), the second linkage member (392) having a first lateral end and a second lateral end that is each offset from the second linkage member axis (398), wherein a first portion of the second linkage member (392) is pivotably coupled to a second portion of the hub (316) such that the first portion of the second linkage member (392) pivots about an axis (32, 340) that is offset from and parallel to the hub axis (318); and
a second blade (400) extending from a proximal end (306, 320, 326, 344, 394, 418, 440, 448, 452, 454, 464, 470) to a distal end (160, 308, 320, 322, 328, 346, 396, 420, 442, 450, 456, 466, 472) along a second blade axis (402), the second blade (400) having a first lateral end and a second lateral end that is each offset from the second blade axis (402), wherein:
(a) a first portion of the second blade (400) is pivotably coupled to second portion of the second linkage member (392) that is disposed at or adjacent to the second lateral end of the second linkage member (392) such that the first portion of the second blade (400) pivots about an axis (32, 340) that is parallel to the hub axis (318), and
(b) a second portion of the second blade (400) is pivotable about a second blade pivot axis (434) that is fixed relative to the base plate (302, 304), the second blade pivot axis (434) being disposed between the first lateral end of the second blade (400) and the second lateral end of the second blade (400) such that (1) when the hub (316) rotates about the hub axis (318) from the first hub (316) position to the second hub (316) position, the second blade (400) rotates about the second blade pivot axis (434) from a second blade (400) closed position to a second blade (400) open position, and (2) when the hub (316) rotates about the hub axis (318) from the second hub (316) position to the first hub (316) position, the second blade (400) rotates about the second blade pivot axis (434) from the second blade (400) open position to the second blade (400) closed position.

2. The cardiac retractor assembly (302) of claim 1, wherein the first lateral edge (312, 332, 350, 404, 422) of the base plate (302, 304) is parallel to the base axis (310).

3. The cardiac retractor assembly (302) of claim 1, wherein the first lateral edge (312, 332, 350, 404, 422) of the baseplate and the second lateral edge (314, 334, 352, 406, 424) of the base plate (302, 304) are parallel to the base axis (310)

4. The cardiac retractor assembly (302) of any one of claims 1 to 3, wherein the distal end (160, 308, 320, 322, 328, 346, 396, 420, 442, 450, 456, 466, 472) of the hub (316) extends up to or distally beyond the distal end (160, 308, 320, 322, 328, 346, 396, 420, 442, 450, 456, 466, 472) of the base plate (302, 304), and the proximal end (306, 320, 326, 344, 394, 418, 440, 448, 452, 454, 464, 470) of the hub (316) extends up to or proximally beyond the proximal end (306, 320, 326, 344, 394, 418, 440, 448, 452, 454, 464, 470) of the base plate (302, 304).

5. The cardiac retractor assembly (302) of any one of claims 1 to 4, wherein a distal end (160, 308, 320, 322, 328, 346, 396, 420, 442, 450, 456, 466, 472) bracket is coupled to or disposed at the distal end (160, 308, 320, 322, 328, 346, 396, 420, 442, 450, 456, 466, 472) of the base plate (302, 304), and a proximal end (306, 320, 326, 344, 394, 418, 440, 448, 452, 454, 464, 470) bracket is coupled to or disposed at the proximal end (306, 320, 326, 344, 394, 418, 440, 448, 452, 454, 464, 470) of the base plate (302, 304),
wherein the second portion of the first blade (342) is pivotably coupled to a first axle (374) that extends along the first blade pivot axis (373) from a first portion of the proximal end (306, 320, 326, 344, 394, 418, 440, 448, 452, 454, 464, 470) bracket to a first portion of the distal end (160, 308, 320, 322, 328, 346, 396, 420, 442, 450, 456, 466, 472) bracket, and the second portion of the second blade (400) is pivotably coupled to a second axle (382) that extends along the second blade pivot axis (434) from a second portion of the proximal end (306, 320, 326, 344, 394, 418, 440, 448, 452, 454, 464, 470) bracket to a second portion of the distal end (160, 308, 320, 322, 328, 346, 396, 420, 442, 450, 456, 466, 472) bracket.

6. A medical device comprising,
a cardiac retractor assembly (302), comprising:
a base plate (302, 304) extending from a proximal end (306, 320, 326, 344, 394, 418, 440, 448, 452, 454, 464, 470) to a distal end (160, 308, 320, 322, 328, 346, 396, 420, 442, 450, 456, 466, 472) along a base axis (310), the base plate (302, 304) having a first lateral edge (312, 332, 350, 404, 422) and a second lateral edge (314, 334, 352, 406, 424) that are each offset from the base axis (310);
an elongated hub (316) that extends along a hub axis (318) from a proximal end (306, 320, 326, 344, 394, 418, 440, 448, 452, 454, 464, 470) to a distal end (160, 308, 320, 322, 328, 346, 396, 420, 442, 450, 456, 466, 472), the hub (316) being rotatably coupled to the base plate (302, 304) such that the hub (316) rotates about the hub axis (318) relative to the base plate (302, 304) from a first hub (316) position to a second hub (316) position;
a first linkage member (324) extending from a proximal end (306, 320, 326, 344, 394, 418, 440, 448, 452, 454, 464, 470) to a distal end (160, 308, 320, 322, 328, 346, 396, 420, 442, 450, 456, 466, 472) along a first linkage member axis (330), the first linkage member (324) having a first lateral end and a second lateral end that is each offset from the first linkage member axis (330), wherein a first portion of the first linkage member (324) is pivotably coupled to a first portion of the hub (316) such that the first portion of the first linkage member (324) pivots about an axis (32, 340) that is offset from and parallel to the hub axis (318);
a first blade (342) extending from a proximal end (306, 320, 326, 344, 394, 418, 440, 448, 452, 454, 464, 470) to a distal end (160, 308, 320, 322, 328, 346, 396, 420, 442, 450, 456, 466, 472) along a first blade axis (348), the first blade (342) having a first lateral end and a second lateral end that is each offset from the first blade axis (348), wherein:
(a) a first portion of the first blade (342) is pivotably coupled to second portion of the first linkage member (324) that is disposed at or adjacent to the second lateral end of the first linkage member (324) such that the first portion of the first blade (342) pivots about an axis (32, 340) that is parallel to the hub axis (318); and
(b) a second portion of the first blade (342) is pivotable about a first blade pivot axis (373) that is fixed relative to the base plate (302, 304), the first blade pivot axis (373) being disposed between the first lateral end of the first blade (342) and the second lateral end of the first blade (342) such that (1) when the hub (316) rotates about the hub axis (318) from the first hub (316) position to the second hub (316) position, the first blade (342) rotates about the first blade pivot axis (373) from a first blade (342) closed position to a first blade (342) open position, and (2) when the hub (316) rotates about the hub axis (318) from the second hub (316) position to the first hub (316) position, the first blade (342) rotates about the first blade pivot axis (373) from the first blade (342) open position to the first blade (342) closed position;
a second linkage member (392) extending from a proximal end (306, 320, 326, 344, 394, 418, 440, 448, 452, 454, 464, 470) to a distal end (160, 308, 320, 322, 328, 346, 396, 420, 442, 450, 456, 466, 472) along a second linkage member axis (398), the second linkage member (392) having a first lateral end and a second lateral end that is each offset from the second linkage member axis (398), wherein a first portion of the second linkage member (392) is pivotably coupled to a second portion of the hub (316) such that the first portion of the second linkage member (392) pivots about an axis (32, 340) that is offset from and parallel to the hub axis (318); and
a second blade (400) extending from a proximal end (306, 320, 326, 344, 394, 418, 440, 448, 452, 454, 464, 470) to a distal end (160, 308, 320, 322, 328, 346, 396, 420, 442, 450, 456, 466, 472) along a second blade axis (402), the second blade (400) having a first lateral end and a second lateral end that is each offset from the second blade axis (402), wherein:
(a) a first portion of the second blade (400) is pivotably coupled to second portion of the second linkage member (392) that is disposed at or adjacent to the second lateral end of the second linkage member (392) such that the first portion of the second blade (400) pivots about an axis (32, 340) that is parallel to the hub axis (318); and
(b) a second portion of the second blade (400) is pivotable about a second blade pivot axis (434) that is fixed relative to the base plate (302, 304), the second blade pivot axis (434) being disposed between the first lateral end of the second blade (400) and the second lateral end of the second blade (400) such that (1) when the hub (316) rotates about the hub axis (318) from the first hub (316) position to the second hub (316) position, the second blade (400) rotates about the second blade pivot axis (434) from a second blade (400) closed position to a second blade (400) open position, and (2) when the hub (316) rotates about the hub axis (318) from the second hub (316) position to the first hub (316) position, the second blade (400) rotates about the second blade pivot axis (434) from the second blade (400) open position to the second blade (400) closed position;
a shaft (196) extending from a proximal end (306, 320, 326, 344, 394, 418, 440, 448, 452, 454, 464, 470) to a distal end (160, 308, 320, 322, 328, 346, 396, 420, 442, 450, 456, 466, 472) along a shaft axis (444), wherein the distal end (160, 308, 320, 322, 328, 346, 396, 420, 442, 450, 456, 466, 472) of the shaft (196) is coupled to the proximal end (306, 320, 326, 344, 394, 418, 440, 448, 452, 454, 464, 470) of the base plate (302, 304), the shaft (196) having one or more interior surfaces that define a shaft (196) interior portion;
an elongated linkage member that extends from a proximal end (306, 320, 326, 344, 394, 418, 440, 448, 452, 454, 464, 470) to a distal end (160, 308, 320, 322, 328, 346, 396, 420, 442, 450, 456, 466, 472) along a linkage member axis (32, 340), the distal end (160, 308, 320, 322, 328, 346, 396, 420, 442, 450, 456, 466, 472) of the linkage member being coupled to the proximal end (306, 320, 326, 344, 394, 418, 440, 448, 452, 454, 464, 470) of the hub (316) such that (1) a rotation of the linkage member in a first rotational direction (206) about the linkage member axis (32, 340) results in the hub (316) rotating about the hub axis (318) from the first hub (316) position to the second hub (316) position and (2) a rotation of the linkage member in a second rotational direction (206) about the linkage member axis (32, 340) results in the hub (316) rotating about the hub axis (318) from the second hub (316) position to the first hub (316) position;
a rotational drive assembly comprising:
a drive housing (478, 506) coupled to the proximal end (306, 320, 326, 344, 394, 418, 440, 448, 452, 454, 464, 470) of the shaft (196);
a screw drive gear that is rotatably coupled to a portion of the drive housing (478, 506), the screw drive gear operatively engaging one or more teeth of a transmission gear that is coupled to the proximal end (306, 320, 326, 344, 394, 418, 440, 448, 452, 454, 464, 470) of the linkage member such that (1) a rotation of the screw drive gear in a first rotational direction (206) results in a rotation of the linkage member in the first rotational direction (206) and (2) a rotation of the screw drive gear in a second rotational direction (206) results in a rotation of the linkage member in the second rotational direction (206).

7. The medical device of claim 6, wherein the shaft (196) is a shaft (196) assembly, the shaft (196) assembly comprising:
a first shaft portion (438, 446) extending along the shaft axis (444) from a distal end (160, 308, 320, 322, 328, 346, 396, 420, 442, 450, 456, 466, 472) to a proximal end (306, 320, 326, 344, 394, 418, 440, 448, 452, 454, 464, 470); and
a second shaft portion (452) extending along the shaft axis (444) from a distal end (160, 308, 320, 322, 328, 346, 396, 420, 442, 450, 456, 466, 472) to a proximal end (306, 320, 326, 344, 394, 418, 440, 448, 452, 454, 464, 470);
wherein the distal end (160, 308, 320, 322, 328, 346, 396, 420, 442, 450, 456, 466, 472) of the first shaft portion (438, 446) is the distal end (160, 308, 320, 322, 328, 346, 396, 420, 442, 450, 456, 466, 472) of the shaft (196) assembly and the proximal end (306, 320, 326, 344, 394, 418, 440, 448, 452, 454, 464, 470) of the second shaft portion (452) is the proximal end (306, 320, 326, 344, 394, 418, 440, 448, 452, 454, 464, 470) of the shaft (196) assembly; and
wherein the first shaft portion (438, 446) is displaceable relative to the first shaft portion (438, 446) such that all or a portion of the first shaft portion (438, 446) is configured to be disposed in an interior portion of the second shaft portion (452) such that the length of the shaft (196) assembly is adjustable.

8. The medical device of claim 6 or 7, wherein the shaft axis (444) is parallel to or coaxially-aligned with the base axis (310).
